# EUROPEAN PATENT APPLICATION

(11) **EP 4 238 988 A1**
(43) Date of publication of application: **06.09.2023**
(21) Application number: 22382180.2
(22) Date of filing: 01.03.2022
(51) Int. Cl.: C07K 16/10, A61P 31/14, A61K 39/395, C12N 15/13, G01N 33/569, G01N 33/577

(54) **ANTIBODIES AGAINST SARS-COV-2 AND USES THEREOF**

(71) Applicant: Consejo Superior De Investigaciones Científicas, 28006 Madrid (ES)
(72) Inventor: SANTIAGO HERNÁNDEZ, César Augusto, E-28006 Madrid (ES); BLANCO LAVILLA, Esther, E-28006 Madrid (ES); ALMAZÁN TORAL, Fernando, E-28006 Madrid (ES); KREMER BARÓN, Leonor, E-28006 Madrid (ES); VARAS RÍOS, Antonio Jesús, E-28006 Madrid (ES); RODRÍGUEZ AGUIRRE, Mª Dolores, E-28006 Madrid (ES); RODRÍGUEZ AGUIRRE, José Francisco, E-28006 Madrid (ES); RODRÍGUEZ FERNÁNDEZ - ALBA, Juan Ramón, E-28006 Madrid (ES)
(74) Representative: ABG Intellectual Property Law, S.L.

(57) **Abstract**

The present invention relates to an antibody which specifically recognizes the receptor binding domain (RBD) of the SARS-CoV-2 spike protein (S) as well as to nucleic acids encoding the antibody thereof. In addition, the invention relates to the use of the antibody of the invention in medicine, more particularly in the treatment of a disease caused by a coronovirus comprising the RBD of the SARS-CoV-2 spike protein. Finally, the invention also relates to a method of detecting the RBD of SARS-CoV-2 spike protein, to a method of diagnosis a coronavirus infection and to a screening or diagnostic kit comprising the antibody of the invention.

## Description

### FIELD OF THE INVENTION

The present invention is comprised within the field of biomedicine. It specifically relates to antibodies which specifically binds to the receptor binding domain (RBD) of the SARS-CoV-2 spike protein and their uses in diagnosis and treatment of infectious diseases.

### BACKGROUND OF INVENTION

Severe acute respiratory syndrome (SARS) is a Coronavirus (CoV) mediated respiratory disease which was first observed in 2002. Based on scientific reports it is assumed that all human CoVs may be of zoonotic origin. Once a human becomes infected, the virus can quickly spread via droplet transmission and close contact between humans, leading to epidemic scenarios or even to a pandemic.

As one example, "Coronavirus Disease 2019" (COVID-19) is caused by the pathogenic coronavirus SARS-CoV-2. Beginning in December 2019, the virus spread globally within a few weeks and led to an international health emergency. The worldwide pandemic poses huge challenges to health systems and leads additionally to restrictions in social life and weakening of global market economies. Since no effective therapy is available to date, and the disease is associated with high morbidity and mortality, there is a great need for therapeutic interventions for those who are ill as well as for prophylactic measures to contain outbreaks.

From a pathophysiological point of view, SARS is a complex medical condition, where the virus starts replicating in the upper respiratory tract and can spread to the lower respiratory tract or target non-respiratory organs and cells. Typical symptoms can be fever, chills, dry cough, dyspnea and diarrhea, although symptoms may first appear 10-14 days post-infection. Clinical investigations show that also liver, kidney, heart, intestine, brain and lymphocytes can, in addition to the lung, be affected.

It is proposed that the virus directly promotes cell damage, whereby a systemic inflammatory response followed by multiple organ injury is triggered. CoVs also cause a dysfunctional renin-angiotensin system, which increases the pulmonary vascular permeability being a factor for the development pulmonary edema. Both systemic inflammatory responses and a dysfunctional renin-angiotensin system contribute to a socalled cytokine storm that triggers an acute respiratory distress syndrome (ARDS). Multi-organ failure and/or the onset of ARDS represent a severe state of SARS patients with a high mortality risk within few weeks or days.

The entry of SARS-CoV-2 into an infected cell is mediated by binding of the viral spike protein via its receptor binding domain (RBD) to the human angiotensin converting enzyme-2 (ACE2) target receptor. Blocking this interaction by human antibodies leads to a neutralization of the virus in patients and thus to a healing of the infection (Hoffmann 2020, Cell. 2020 Apr 16; 181(2): 271-280.e8).

Neutralizing antibodies against SARS-CoV-2 have been described previously (Jiang 2020). Virus neutralizing antibodies play crucial roles in controlling viral infection. Currently developed SARS-CoV- and MERS-CoV-specific Abs target S1-RBD, S1-NTD, or the S2 region, blocking the binding of RBDs to their respective receptors and interfering with S2-mediated membrane fusion or entry into the host cell, thus inhibiting viral infections.

Therefore, various SARS-CoV- and MERS-CoV-mAbs have been described in the art in the context of their potential cross-neutralizing activity against SARS-CoV-2 infection. Very recently, further reports on anti-CoV-Spike antibodies isolated from convalescent patients post COVID-19 disease are also appearing, although additional research is required to assess these early reports. Other human monoclonal SARS-CoV-2 neutralizing antibodies, chimeric antibodies or single-domain antibodies have been disclosed in the art (Wang et al, 2020, Clin Infect Dis, 17;71(10):2688-2694; Wu Yanling et al, 2020, Cell Host Microbe, 2020, 27(6):891-898.e5). Upon review of the antibodies disclosed in these preliminary reports, it appears that one or more of the antibody properties, with respect to plaque reduction neutralization (as shown in a PRNT), affinity of the antibodies to the RBD epitope, an inhibition of ACE2 binding to Spike protein, potential unspecific cross reaction to other tissue types, the in vivo properties of the antibodies, for example in animal models of SARS-CoV-2 infection and COVID disease, or the ability to bind and neutralize multiple SARS-CoV-2 variants or mutants, are suboptimal.

The present application is directed to overcoming deficiencies in the art related with the treatment and diagnosis of the infectious disease.

### SUMMARY OF THE INVENTION

The authors of the present invention have isolated mice sera containing polyclonal antibodies capable of selectively bind to the SARS-CoV-2 spike protein. More particularly, the disclosed monoclonal antibody (mAb) binds to a conformational epitope located in the receptor binding domain (RBD) of the SARS-CoV-2 spike protein and have a neutralizing capacity against SARS-CoV-2 much higher than other known commercially available monoclonal anti-SARS-CoV-2 antibody.

Thus, in a first aspect the invention relates to an antibody which specifically recognizes the receptor binding domain (RBD) of the SARS-CoV-2 spike protein, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

In a second aspect the invention relates to the nucleic acids encoding the VH and/or VL of the antibody of the invention as well as vectors and host cells.

In a third aspect, the invention relates to the antibody, nucleic acids, vectors and host cells of the invention for use in medicine.

In a fourth aspect, the invention relates to the antibody, nucleic acids, vectors host cells of the invention for use in a method of treatment of a disease caused by a coronavirus comprising the RBD of the SARS-CoV-2 spike protein or an immunologically equivalent variant thereof.

In a fifth aspect, the invention relates to a pharmaceutical composition comprising an effective amount of the antibody, nucleic acids, expression vectors or host cells of the invention and a pharmaceutically acceptable carrier.

In a sixth aspect, the invention relates to the pharmaceutical composition of the invention for use in in the treatment of disease caused by SARS-CoV-2.

In a seventh aspect, the invention relates to a method of detecting the presence of the RBD of SARS-CoV-2 spike protein or an immunologically equivalent variant thereof in a sample, the method comprising:
a) contacting the sample with the antibody of the invention, wherein said contacting is carried out under non-reducing conditions; and
b) detecting the formation of a complex containing the antibody and the RBD of SARS-CoV-2 spike protein or an immunologically equivalent variant thereof,
wherein the detection of the complex indicates the presence of the RBD of SARS-CoV-2 spike protein or an immunologically equivalent variant thereof in the sample.

In an eighth aspect, the invention relates to a method of diagnosing a coronavirus infection in a subject, wherein the coronavirus comprises the RBD of the SARS-CoV-2 spike protein or an immunologically equivalent variant thereof, the method comprising detecting the presence of the RBD of the SARS-CoV-2 spike protein or an immunologically equivalent variant thereof in a sample from the subject by the method of the seventh aspect of the invention, wherein the detection of the RBD of the SARS-CoV-2 spike protein or immunologically equivalent thereof in the sample is indicative that the patient suffers an infection of a coronavirus which comprises the RBD of the SARS-CoV-2 spike protein or an immunologically equivalent variant thereof.

In a final aspect, the invention relates to a diagnostic kit or a screening kit comprising the antibody of the invention.

### DESCRIPTION OF THE FIGURES

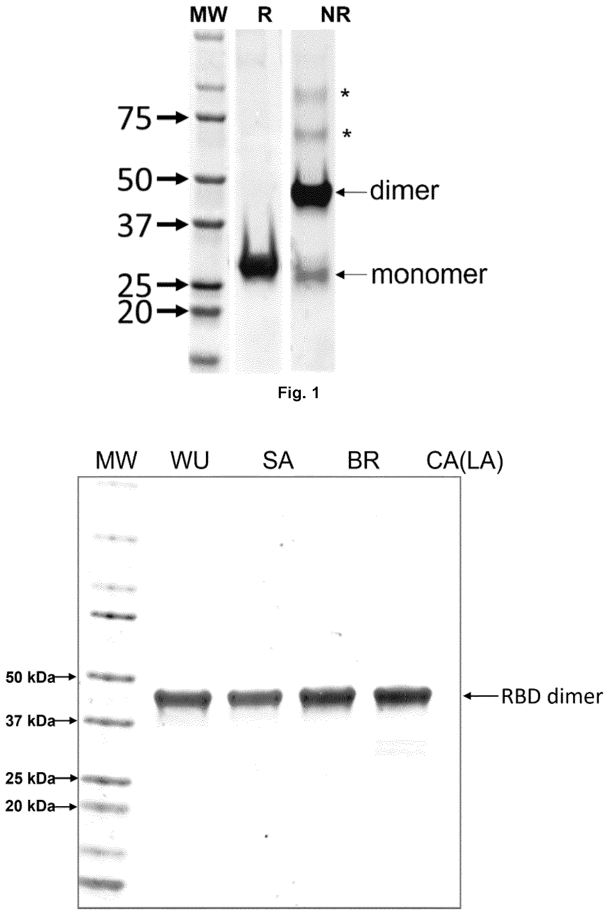

**Figure 1****.** Analysis by SDS-PAGE of the IMAC purified RBD protein. The purified recombinant protein (2 µg), corresponding to Wuham variant of SARS-CoV-2 was analyzed by SDS-PAGE (4% -15% gradient) under non-reducing conditions NR (sample buffer without β-mercaptoethanol), or under reducing conditions R (sample buffer containing β-mercaptoethanol). After that, proteins were visualized by Coomassie-blue staining. Standard molecular weight markers (BioRad) are shown on the left (MW).
**Figure 2****.** Analysis by SDS-PAGE of the different purified RBD recombinant proteins. The dimeric forms of the purified recombinants proteins (1 µg of each protein), corresponding to Wuham (WU), South Africa (SA), Brazil (BR) or California [CA(LA)] variants of SARS-CoV-2 were analyzed by SDS-PAGE (4% -15% gradient) under non-reducing conditions (sample buffer without β-mercaptoethanol). After that, proteins were visualized by Coomassie-blue staining. Standard molecular weight markers (BioRad) are shown on the left (MW).

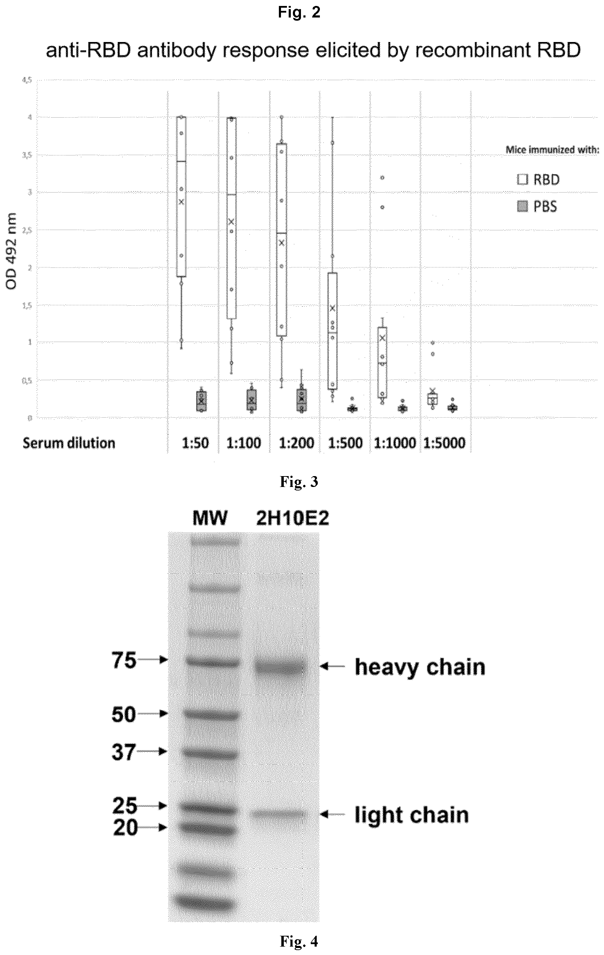

**Figure 3****.** Anti-RBD antibody response elicited by recombinant RBD. 96 wells ELISA plates were coated overnight with purified recombinant RBD (Wuhan) diluted in PBS (1 µg/mL). After that, wells were washed three times with PBS, and later incubated with blocking solution (PBS-casein [1%], BioRad) at room temperature (RT) for 1 h. Then, wells were washed again, and incubated (1h at RT) with serial dilutions (1:50 to 1:5000) of mice sera. After that, wells were washed again 5 times, and incubated (1h at RT) with rabbit-anti mouse IgG-HRP (Abcam). After washing wells 5 times, they were incubated with OPD solution for 15 min. Then, the reaction was stopped by addition of H₂SO₄ (3M). Absorbance at 492 nm was measured in a spectrophotometer.
**Figure 4****.** Analysis by SDS-PAGE purified mAb 2H10E2. Purified mAb 2H10E2 (1 µg), was analyzed by SDS-PAGE (4% -15% gradient) under reducing conditions (sample buffer containing β-mercaptoethanol). After that, proteins were visualized by Coomassie-blue staining. Standard molecular weight markers (BioRad) are shown on the left (MW).

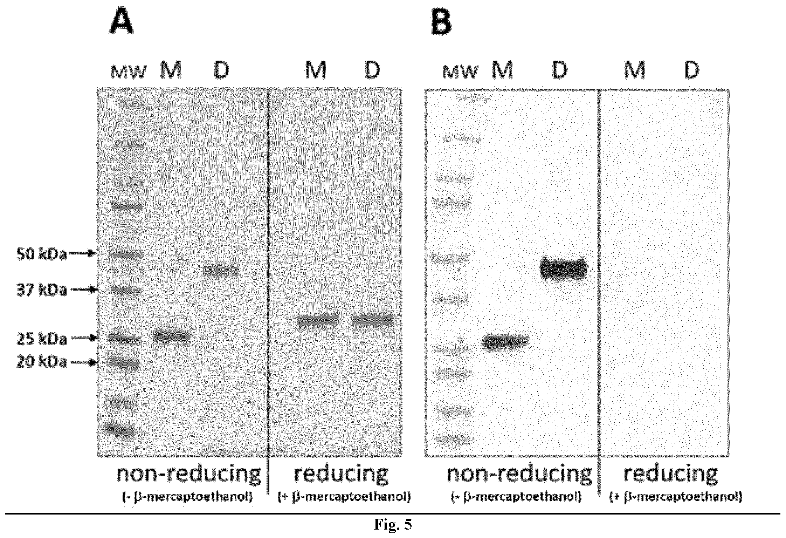

**Figure 5****.** Characterization of mAb 2H10E2 by Western-blot. A) Purified monomers (M) or dimers (D) of RBD (1 µg), were analyzed by SDS-PAGE (4% -15% gradient) under non-reducing conditions (left part of panel A), or under reducing conditions (right part of panel A). After that, proteins were visualized by Coomassie-blue staining. B) A similar SDS-PAGE analysis was repeated, and proteins were transferred to a nitrocellulose membrane. This membrane was incubated for 1h at 37°C, with a 1:2000 diluted mAb 2H10E2, washed three times with TBD-tween 0.5%, and incubated with peroxidase-conjugated goat anti-mouse Ig secondary antibody (dilution 1:2000 in bloto 5%), and bound antibodies were detected using a solution of chloronaphtol plus H₂O₂. Standard molecular weight markers (BioRad) are shown on the left (MW).

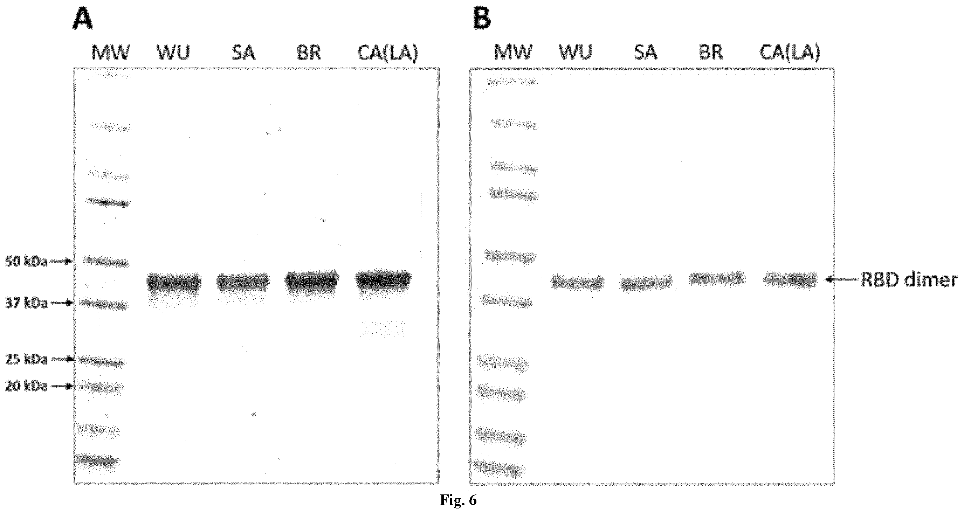

**Figure 6****.** mAb 2H10E2 recognizes RBD dimers from different SARS-CoV-2 variants. A) Purified dimers of the purified recombinants proteins (1 µg of each protein), corresponding to Wuham (WU), South Africa (SA), Brazil (BR) or California [CA(LA)] variants of SARS-CoV-2 were analyzed by SDS-PAGE (4% -15% gradient) under non-reducing conditions (sample buffer without β-mercaptoethanol). After that, proteins were visualized by Coomassie-blue staining. B) A similar SDS-PAGE analysis was repeated, and proteins were transferred to a nitrocellulose membrane. This membrane was incubated for 1h at 37°C, with a 1:2000 diluted mAb 2H10E2, washed three times with TBD-tween 0.5%, and incubated with peroxidase-conjugated goat anti-mouse Ig secondary antibody (dilution 1:2000 in bloto 5%), and bound antibodies were detected using a solution of chloronaphtol plus H₂O₂. Standard molecular weight markers (BioRad) are shown on the left (MW).

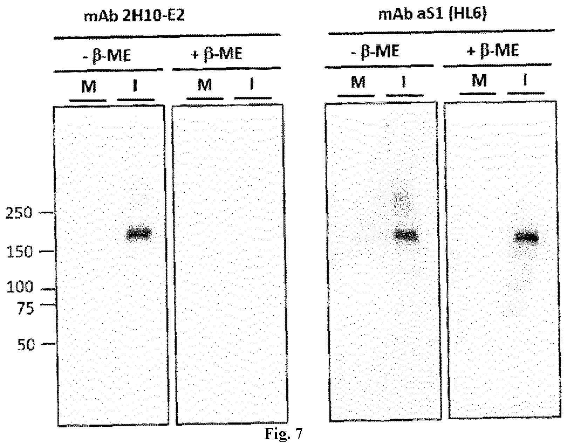

**Figure 7****.** mAb of 2H10E2 recognizes spike protein expressed in SARS-CoV-2-infected cells by Western-blot. Extracts from VERO E6 cells infected (I) or non-infected (M) with SARS-CoV-2 at an moi of 1 PFU/cell were collected at 18 hpi and analyzed by Western-blot under reducing conditions (+ β-ME) and not reducing (- β-ME) with mAb 2H10E2 (diluted 1: 2000) or a commercial mouse anti-S antibody (Diluted 1: 2500; Genetex, GTX632604) as a positive control [aS1 (SL6)]. Molecular weights in kDa, are denoted at the left of the Figure.

### DETAILED DESCRIPTION OF THE INVENTION

The authors of the invention disclose herewith new neutralizing antibodies against SARS-CoV-2 and related virus.

Unless otherwise defined, all technical terms used herein have the same meaning as commonly understood by one of ordinary skill in the art to which this invention belongs. The definitions provided herewith and in every other aspect of the invention are equally applicable to the whole invention.

### Antibodies

In a first aspect the invention relates to an antibody which specifically recognizes the receptor binding domain (RBD) of the SARS-CoV-2 spike protein, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

The term "antibody", as used herein, refers to a molecule which has the ability to specifically bind to an epitope of a molecule ("antigen"). Naturally occurring antibodies typically comprise a tetramer which is usually composed of at least two heavy (H) chains and at least two light (L) chains. Each heavy chain (50-75 KDa) is comprised of a heavy chain variable domain (abbreviated herein as VH) and a heavy chain constant domain, usually comprised of three domains (CH1, CH2 and CH3). Heavy chains can be of any isotype, including α, δ, γ, ε, µ, which corresponds to five antibodies isotypes, IgA (IgA1 and IgA2), IgD, IgG (lgG1, IgG2, IgG3 and IgG4 subtypes), IgD, and IgM. Each light chain (25 KDa) is comprised of a light chain variable domain (abbreviated herein as VL) and a light chain constant domain (CL). Light chains include kappa chains and lambda chains. The heavy and light chain variable domain is typically responsible for antigen recognition, while the heavy and light chain constant domain may mediate the binding of the immunoglobulin to host tissues or factors, including various cells of the immune system (e.g., effector cells) and the first component (C1 q) of the classical complement system. The VH and VL domains can be further subdivided into domains of hypervariability, termed "complementarity determining regions" that are interspersed with domains of more conserved sequence, termed "framework regions" (FR). Each VH and VL is composed of three CDR domains and four FR domains arranged from amino-terminus to carboxy-terminus in the following order: FR1 -CDR1 -FR2- CDR2-FR3-CDR3-FR4. The term "complementarity determining region" or "CDR", as used herein, refers to the region within an antibody where this protein complements an antigen's shape. Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy chain and the light chain are characterized by three CDRs, respectively CDRH1, CDRH2, CDRH3 and CDRL1, CDRL2, CDRL3.

The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue: W34-40), by following the numbering provided by Kabat et al, Sequences of Proteins of Immunological Interest, 5th ed., Public Health Service, National Institutes of Health, Bethesda, Md. (1991), or by following the numbering provided by Chothia et al. (1989, Nature 342:877-83).

As used herein, the antibody of the invention encompasses not only full length antibodies (e.g., IgG), but also antigen-binding fragments thereof, for example, Fab, Fab', F(ab')₂, Fv fragments, human antibodies, humanised antibodies, chimeric antibodies (including bispecific antibodies), antibodies of a non-human origin, recombinant antibodies, and polypeptides derived from immunoglobulins produced by means of genetic engineering techniques, for example, single chain Fv (scFv), diabodies, heavy chain or fragments thereof, light chain or fragment thereof, V_{H} or dimers thereof, V_{L} or dimers thereof, Fv fragments stabilized by means of disulfide bridges (dsFv), molecules with single chain variable region domains (Abs), minibodies, scFv-Fc, VL and VH domains and fusion proteins comprising an antibody, or any other modified configuration of the immunoglobulin molecule that comprises an antigen recognition site of a desired specificity. The antibody of the invention may also be a bispecific antibody. An antibody fragment may refer to an antigen binding fragment.

As used herein a "recombinant antibody" is an antibody that comprises an amino acid sequence derived from two different species or two different sources, and includes synthetic molecules, for example, an antibody that comprises a non-human CDR and a human framework or constant region. In certain embodiments, recombinant antibodies of the present invention are produced from a recombinant DNA molecule or synthesized.

Of particular relevance are antibodies and their epitope- binding fragments that have been "isolated" so as to exist in a physical milieu distinct from that in which it may occur in nature or that have been modified so as to differ from a naturally occurring antibody in amino acid sequence.

The term "antibody" comprises whole monoclonal antibodies or polyclonal antibodies, or fragments thereof, that retain one or more CDR regions, and includes human antibodies, humanised antibodies, chimeric antibodies and antibodies of a non-human origin.

"Monoclonal antibodies (mAbs)" are homogenous, highly specific antibody populations directed against a single site or antigenic "determinant". "Polyclonal antibodies (pAbs)" include heterogeneous antibody populations directed against different antigenic determinants.

In a particular embodiment, the antibody of the invention is an antibody of non-human origin, preferably of murine origin. In preferred embodiment, the antibody of the invention is a monoclonal antibody.

In a particular embodiment, the antibody of the invention is of the type IgM heavy chain and kappa light chain. In another embodiment, the antibody of the invention is of the type IgM heavy chain and lambda light chain. In another embodiment, the antibody of the invention is of the type IgG heavy chain and kappa light chain. In another embodiment, the antibody of the invention is of the type IgG heavy chain and lambda kappa chain.

In a preferred embodiment, the antibody of the invention is a chimeric antibody. In a still preferred embodiment chimeric antibody a human-mouse chimeric antibody which comprises mouse variable domains (Y_{λ}, V_{κ}, and V_{H}) linked to human constant domains (C_{κ} and CH1), in particular mouse V_{λ}/V_{κ} domains fused to human C_{κ} and mouse V_{H} domain fused to human CH1 of human IgG₁.

The person skilled in the art will understand that the amino acid sequences of the antibodies of the invention can include one or more amino acid substitutions such that, even though the primary sequence of the polypeptide is altered, the capacity of the antibody to bind to receptor binding domain (RBD) of the SARS-CoV-2 spike protein is maintained. Said substitution can be a conservative substitution and is generally applied to indicate that the substitution of one amino acid with another amino acid with similar properties (for example, the substitution of glutamic acid (negatively charged amino acid) with aspartic acid would be a conservative amino acid substitution).

Amino acid sequence modification(s) of the antibody described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antibody. Any combination of deletion, insertion, and substitution is made to achieve the final construct, provided that the final construct possesses the desired characteristics. The amino acid changes may also alter post-translational processes of the protein, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

Another type of variant is an amino acid substitution variant. These variants have at least one amino acid residue in the molecule replaced by a different residue. The sites of greatest interest for substitution mutagenesis of antibodies include the hypervariable regions, but FR alterations are also contemplated.

Another type of amino acid variant of the antibody alters the original glycosylation pattern of the antibody. By altering is meant deleting one or more carbohydrate moieties found in the molecule, and/or adding one or more glycosylation sites that are not present in it. Glycosylation of polypeptides is typically either N-linked or O-linked. N-linked refers to the attachment of the carbohydrate moiety to the side chain of an asparagine residue. The tripeptide sequences Asparagine-X-Serine and Asparagine-X-Threonine, where X is any amino acid except proline, are the recognition sequences for enzymatic attachment of the carbohydrate moiety to the asparagine side chain. Thus, the presence of any of these tripeptide sequences in a polypeptide creates a potential glycosylation site. O-linked glycosylation refers to the attachment of one of the monosaccharides or monosaccharide derivatives N-aceylgalactosamine, galactose, or xylose to a hydroxyamino acid, most commonly serine or threonine, although 5-hydroxyproline or 5 -hydroxy lysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites). Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non- variant version of the antibody.

The affinity of an antibody for an antigen can be defined as the effectiveness of the antibody for binding such antigen. Antigen-antibody binding is a reversible binding and so, when both molecules are in dilution in the same solution after sufficient time, this solution reaches an equilibrium in which the concentrations of antigen-antibody complex (AgAb), free antigen (Ag) and free antibody (Ab) are constant. Therefore, the ratio [AgAb]/[Ag]*[Ab] is also a constant defined as association constant named Ka which can be used to compare the affinity of some antibodies for its respective epitope.

The common way to measure the affinity is to experimentally determine a binding curve. This involves measuring the amount of antibody-antigen complex as a function of the concentration of the free antigen. There are two common methods of performing this measurement: (i) the classical equilibrium dialysis using Scatchard analysis and (ii) the surface plasmon resonance method in which either antibody or antigen are bound to a conductive surface and binding of antigen or antibody respectively affects the electrical properties of this surface.

The capacity of the antibody of the invention to bind to the receptor binding domain (RBD) of the SARS-CoV-2 spike protein can be determined by a number of assays that are available in the art. Preferably, the binding specificity of monoclonal antibodies produced by a clone of hybridoma cells is determined by immunoprecipitation or by an in vitro binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry.

Often it is only necessary to determine relative affinities of two or more antibodies that join the same epitope, such as in the case of the antibody of the invention and a functional variant thereof. In this case a competitive assay can be performed in which a serial dilution of one of the antibodies is incubated with a constant quantity of a ligand, and the second antibody labelled with any suitable tracer is then added. After binding of this mAb and washing the non-bounded antibodies, the concentration of second antibody is measured and plotted in relation to concentrations of the first antibody and analysed with Scatchard method.

The term "recognizes", "binding", "bond" or "binds" are used herein interchangeably and refer to the interaction between affinity binding molecules or specific binding pairs as a result of non-covalent bonds, such as, but not limited to, hydrogen bonds, hydrophobic interactions, van der Waals bonds, ionic bonds or a combination of the above. The term "binding pair" does not involve any particular size of any other technical structural characteristic other than that said binding pair can interact and bind to the other member of the binding pair resulting in a conjugate wherein the first and second components are bound to each other by means of the specific interaction between the first and second member of a binding pair. Within the context of the present invention binding pair includes any type of immune interaction such as antigen/antibody or antigen/antibody fragment.

The terms "specifically recognizes" or "specifically binds" are used in the present invention to refer to the binding of an antibody or a fragment thereof to the RBD of the SARS-CoV-2 spike protein, is understood as the capacity of the antibody or fragment thereof to bind specifically to its target, the RBD of the SARS-CoV-2 spike protein, by means of the existence of complementarity between the three-dimensional structures of the two molecules with a substantially higher affinity for non-specific binding such that the binding between said antibody or fragment thereof and the the RBD of the SARS-CoV-2 spike protein preferably takes place before the binding of any of said molecules with respect to the other molecules present in the reaction mixture. This results in that the antibody or fragment thereof does not cross-react with other antigens which may or may not be present in the sample. Cross-reactivity of the antibody or fragment thereof may be tested, for example, by assessing binding of said antibody or fragment thereof under conventional conditions to the glycan of interest as well as to a number of more or less (structurally and/or functionally) closely related peptides. Only if the antibody or fragment thereof binds to the peptide of interest but does not or does not essentially bind to any of the other antigens is considered specific for the peptide of interest. For instance, a binding can be considered specific if the binding affinity between the antibody and the the RBD of the SARS-CoV-2 spike protein has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

The term "KD" or "equilibrium dissociation constant" refers to a ratio of koff/kon, between the antibody and its antigen. KD and affinity are inversely related. The KD value relates to the concentration of antibody (the amount of antibody needed for a particular experiment) and so the lower the KD value (lower concentration) and thus the higher the affinity of the antibody.

The capacity of the binding agents according to the invention, and in particular of the antibody or antibody fragment as described herein, to bind to the RBD of the SARS-CoV-2 spike protein can be determined by a number of assays that are well known in the art. Preferably, the binding capacity of the binding agents is determined by immunoprecipitation or by an *in vitro* binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry.

The CoV Spike protein, also known as S protein, is a glycoprotein trimer, wherein each monomer of the trimeric S protein is about 180 kDa, and contains two subunits, S1 and S2, mediating attachment and membrane fusion, respectively.

In an embodiment, the antibody target is the receptor binding domain (RBD) of the S1 subunit of a recombinant Spike of SARS-CoV-2 (SARS-CoV-2-Spike-S1-RBD). In another particular embodiment, the recombinant SARS-CoV-2-Spike-S1-RBD is produced under non-reducing conditions and therefore the recombinant protein is mainly a homodimer. In another particular embodiment, the recombinant SARS-CoV-2-SpikeS1-RBD is produced in reducing conditions and therefore the recombinant protein is formed by dimers and oligomeric forms.

The term "SARS-CoV-2-Spike-S1-RBD" as used herein refers the amino acid sequence of amino acid residues 319-541 of the S protein of the SARS-CoV-2. The S protein of the SARS-CoV-2-spike protein refers to the S protein of the Wuhan (MN908947.3, from 18 March 2020) comprising the sequence identified here as SEQ ID NO: 17. It may also refer to the amino acid sequence of amino acid residues 319-541 of the S protein of the SARS-CoV-2 or the following lineages and variants, wherein the sequence of the RBD region of the S protein
From each variant is shown in brackets:
- Lineage B.1.1.7 / Variant of Concern Alpha (20-DEC-01) (comprising the sequence identified here as SEQ ID NO: 18): First detected in October 2020 during the COVID-19 pandemic in the United Kingdom Lineage B.1.1.7, was previously known as the first Variant Under Investigation in December 2020 (VUI - 202012/01) and later notated as VOC-202012/01. It is also known as lineage B.1.1.7 or 201/501 Y.V1 (formerly 2p.337 0B/501Y.V1). As of May 2021, Lineage B.1 .1.7 has been detected in some 120 countries.
- Variant of Concern 21 FEB-02: Previously written as VOC-202102/02, described by Public Health England (PHE) as "B.1.1.7 with E484K" is of the same lineage in the Pango nomenclature system, but has an additional E484K mutation.
- Lineage B.1.1.207: First sequenced in August 2020 in Nigeria, this variant has a P681 H mutation, shared in common with UK's Lineage B.1 .1 .7. It shares no other mutations with Lineage B.1.1.7 and as of late December 2020 this variant accounts for around 1% of viral genomes sequenced in Nigeria. As of May 2021 , Lineage B.1.1.207 has been detected in 10 countries.
- Lineage B.1.1.317: B.1.1.317 was identified in Queensland, Australia.
- Lineage B.1.1.318 (comprising the sequence identified here as SEQ ID NO: 19): Lineage B.1 .1.318 was designated by PHE as a VUI (VUI-21 FEB-04, previously VU 1-202102/04) on 24 February 2021.
- Lineage B.1.351 (Beta) (comprising the sequence identified here as SEQ ID NO: 20): On 18 December 2020, the 501. V2 variant, also known as 501. V2, 20H/501Y.V2 (formerly 20C/501Y.V2), VOC-20DEC-02 (formerly VOC-202012/02), or lineage B.1.351, was first detected in South Africa and reported by the country's health department. The variant contains several mutations that allow it to attach more easily to human cells because of the following three mutations in the receptor-binding domain (RBD) in the spike glycoprotein of the virus: N501Y, K417N, and E484K.
- Lineage B.1.429 / CAL.20C (Epsilon) (comprising the sequence identified here as SEQ ID NO: 21).
- Lineage B.1.429, also known as CAL.20C, is defined by five distinct mutations (I4205V and D1183Y in the ORF1ab-gene, and S13I, W152C, L452R in the spike protein's S-gene). B.1.429 was first observed in July 2020 by researchers at the Cedars-Sinai Medical Center, California, in one of 1 ,230 virus samples collected in Los Angeles County since the start of the COVID-19 epidemic.
- Lineage B.1.525 (Eta) (comprising the sequence identified here as SEQ ID NO: 19): B.1.525, also called VUI-21 FEB-03[15] (previously VU 1-202102/03) by Public Health England (PHE) and formerly known as UK1188, does not carry the same N501Y mutation found in B.1.1.7, 501. V2 and P.1, but carries the same E484K-mutation as found in the P.1 , P.2, and 501. V2 variants, and also carries the same AH69/AV70 deletion (a deletion of the amino acids histidine and valine in positions 69 and 70) as found in B.1 .1 .7, N439K variant (B. 1.141 and B.1.258) and Y453F variant (Cluster 5). B.1.525 differs from other variants by having both the E484K-mutation and a new F888L mutation (a substitution of phenylalanine (F) with leucine (L) in the S2 domain of the spike protein).
- Lineage B.1.526 (lota) (comprising the sequence identified here as SEQ ID NO: 19): In November 2020, a mutant variant was discovered in New York City, which was named B.1.526.
- Lineage B.1.617.1 (Kappa) (comprising the sequence identified here as SEQ ID NO: 22): In October 2020, a new variant was discovered in India, which was named B.1.617.1. Among some 15 defining mutations, it has spike mutations D111 D (synonymous substitution), G142D, P681 R, E484Q and L452R, the latter two of which may cause it to easily avoid antibodies. Public Health England (PHE) designated B.1.617 as a 'Variant under investigation', VUI-21 APR-01. On 29 April 2021, PHE added two further variants, VUI-21 APR- 02 and VUI-21APR-03, effectively B.1.617.2 and B.1.617.3. B.1.617.2 (which notably lacks mutation at E484Q; Variant Delta), (comprising the sequence identified here as SEQ ID NO: 23) is a "variant of concern".
- Lineage B.1.618: This variant was first isolated in October 2020, and has the E484K mutation as in South African variant B.1.351.
- Lineage B.1.620 (comprising the sequence identified here as SEQ ID NO: 24).
- In March 2021, this variant was discovered in Lithuania.
- Lineage P.1 : Lineage P.1 (comprising the sequence identified here as SEQ ID NO: 25), termed Variant of Concern 21 JAN-02 (formerly VOC-202101/02) (Gamma) by Public Health England and 20J/501Y.V3 by Nextstrain, was detected in Tokyo on 6 January 2021 by the National Institute of Infectious Diseases (NIID). This variant of SARS-CoV-2 has been named in the P.1 lineage, and has 17 unique amino acid changes, 10 of which in its spike protein, including N501Y, E484K and K417T.
- Lineage P.3 (Teta) (comprising the sequence identified here as SEQ ID NO: 26): On 18 February 2021, the Department of Health of the Philippines confirmed the detection of two mutations of COVID-19 in Central Visayas after samples from patients were sent to undergo genome sequencing. The mutations were later named as E484K and N501Y.
- Lineage B.1.1.529 (comprising the sequence identified here as SEQ ID NO: 27) (Omicron), Sudafrica, GISAID clade GRA.
- Lineage C.37 (comprising the sequence identified here as SEQ ID NO: 28), (Lambda) Peru, GISAID clade GR/452Q.V1.
- Lineage B.1.621 (comprising the sequence identified here as SEQ ID NO: 29) (Mu), Colombia, GISAID clade GH.

In an embodiment, the antibody target is the receptor binding domain (RBD) of the S1 subunit (S1) of a recombinant Spike glycoprotein of SARS-CoV-2 (SARS-CoV-2-SpikeS1-RBD glycoprotein). According to the present invention, in some embodiments, the antibody target, namely the receptor binding domain (RBD) of the S1 subunit (S1) of a recombinant Spike glycoprotein of SARS-CoV-2 (SARS-CoV-2-Spike-S1-RBD glycoprotein), is expressed in cells which produce the protein target of the inventive antibodies, the SARS-CoV-2-Spike-S1-RBD in a glycosylated form.

A skilled person can determine and/or predict the glycosylation of a protein form any given cell type. For example, glycosylation patterns can be predicted using software, for example the NetNGlyc 1.0 or 4.0 Server or N-GlyDE, a two-stage N-linked glycosylation site prediction incorporating gapped dipeptides and pattern-based encoding.

In another embodiment, the antibody target is expressed in insect cells. In a more particular embodiment, the insect cells are a cell line originated from the ovarian cells of the cabbage looper Trichoplusia ni., (officially called BTI-Tn-5B1-4).

The SARS-CoV-2 S protein RBD comprises an amino acid sequence of amino acid residues 319-541 of the S protein of the SARS-CoV-2 of any of the variants or lineages above referred or an amino acid sequence having at least 95% sequence identity with amino acid residues 319-541 of the S protein of the SARS-CoV-2. In a preferred embodiment the SARS-CoV-2 S protein or a portion thereof comprises the SARS-CoV-2 S protein RBD having an amino acid sequence having at least 96%, at least 97%, at least 98%, or at least 99% sequence identity with the sequence of amino acid residues 319-541 of the S protein of the SARS-CoV-2. In one embodiment the SARS-CoV-2 S protein RBD has an amino acid sequence having at least 98% to 100% sequence identity with amino acid residues 319-541 of the S protein of the SARS-CoV-2. In a specific embodiment, the COVID-19 coronavirus (SARS-CoV-2) spike (S) protein or a portion thereof is the SARS-CoV-2 S protein RBD having or consisting of an amino acid sequence of amino acid residues 319-541 of the S protein of the SARS-CoV-2 or an amino acid sequence having at least 95% sequence identity with amino acid residues 319-541 of the S protein of the SARS-CoV-2. In a specific embodiment the SARS-CoV-2 S protein RBD may also be the S protein RBD of a variant of SARS-CoV-2, such as lineage B.1.1.7, B.1.351 or P.1.

In another embodiment, the target sequence, that is, the amino acid comprising the amino acid residues 319-541 of the S protein of the SARS-CoV-2, additionally comprises 9 histidine residues (HHHHHHHHH) fused to the N-terminus of the RBD domain of the spike protein.

Within the context of the present invention, the term "at least 95% sequence identity with" refers to a protein that may differ in the amino acid sequence and/or the nucleic acid sequence encoding the amino acid sequence of the reference sequence, such as the amino acid sequence of the S protein of the SARS-CoV-2 or the amino acid sequence of amino acid residues 1-1208, amino acid residues 1-681 or amino acid residues 319-541 of the S protein of the SARS-CoV-2 (also referred to as the corresponding portion thereof). The S protein or the portion thereof may be of natural origin, e.g. a mutant version or a variation of the S protein of SARS-CoV-2 having the amino acid sequence of the S protein of the SARS-CoV-2or an engineered protein, e.g. an engineered glycoprotein derivative, which has been modified by introducing site directed mutations or cloning, or a combination thereof. It is known that the usage of codons is different between species. Thus, when expressing a heterologous protein in a target cell, it may be necessary, or at least helpful, to adapt the nucleic acid sequence to the codon usage of the target cell. Methods for designing and constructing derivatives of a given protein are well known to the person skill in the art. Adapting the nucleic acid sequence to the codon usage of the target cell is also known as codon-optimization.

The term "epitope", as used herein, refers to that portion of a given immunogenic substance that is the target of or is bound by an antibody or a cell-surface receptor of a host immune system that has mounted an immune response to the given immunogenic substance as determined by any method known in the art. Further, an epitope may be defined as a portion of an immunogenic substance that elicits an antibody response or induces a T-cell response in an animal, as determined by any method available in the art. See Walker J, Ed., "The Protein Protocols Handbook" (Humana Press, Inc., Totoma, NJ, US, 1996). The term "epitope" may also be used interchangeably with "antigenic determinant" or "antigenic determinant site". The epitopes of protein antigens are divided into two categories, conformational epitopes and linear epitopes, based on their structure and interaction with the antibody.

The terms "polypeptide" and "peptide" are used interchangeably herein to refer to polymers of amino acids of any length.

The term "amino acid" refers to naturally occurring and synthetic amino acids, as well as amino acid analogs and amino acid mimetics that function in a manner similar to the naturally occurring amino acids. Furthermore, the term "amino acid" includes both D-and L-amino acids (stereoisomers).

The term "complementarity determining region" or "CDR", as used herein, refers to the region within an antibody where this protein complements an antigen's shape. Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy chain and the light chain are characterized by three CDRs, respectively VH-CDR1, VH-CDR2, VH-CDR3 and VL-CDR1, VL-CDR2, VL-CDR3.

As it is used herein, the term "functionally equivalent variant of a CDR sequence" refers to a sequence variant of a particular CDR sequence having substantially similar sequence identity with it and substantially maintaining its capacity to bind to its cognate antigen when being part of an antibody or antibody fragment as the ones described herein. For example, a functionally equivalent variant of a CDR sequence may be a polypeptide sequence derivative of said sequence comprising the addition, deletion or substitution of one or more amino acids.

Functionally equivalent variants of a CDR sequence according to the invention include CDR sequences having at least approximately 70%, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a CDR sequence according to the invention will preferably maintain at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 1 to 6 to bind to its cognate antigen when being part of an antibody or antibody fragment as the ones of the invention. This capacity to bind to its cognate antigen may be determined as a value of affinity, avidity, specificity and/or selectivity of the antibody or antibody fragment to its cognate antigen.

In one embodiment, the VH of the antibody of the invention is characterized in that the CDR1 region
- shows at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence DYNMH (SEQ ID NO: 1);
- at least 1, at least 2, at least 3, at least 4 or all are conservative substitutions of the amino acids found in the corresponding positions in the sequence DYNMH (SEQ ID NO: 1);

In one embodiment, the VH of the antibody of the invention is characterized in that the CDR2 region
- shows at least 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence YIYPYNGGTGYNQKFKS (SEQ ID NO: 2);
- at least 1, at least 2, at least 3, at least 4, at least 5, at least 6, at least 7, at least 8, at least 9, at least 10, at least 11, at least 12, at least 13, at least 14, at least 15, at least 16 or all are conservative substitutions of the amino acids found in the corresponding positions in the sequence YIYPYNGGTGYNQKFKS (SEQ ID NO: 2);

In another embodiment, the VH of the antibody of the invention is characterized in that the CDR3 region
- shows at least 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence AITTDYYAMDY (SEQ ID NO: 3);
- at least 1, at least 2, at least 3, at least 4, at least 5, at least 6, at least 7, at least 8, at least 9, at least 10 or all are conservative substitutions of the amino acids found in the corresponding positions in the sequence AITTDYYAMDY (SEQ ID NO: 3).

In another embodiment, the VL of the antibody of the invention is characterized in that the CDR1 region
- shows at least 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence RASESVDNYGISFMN (SEQ ID NO: 4);
- at least 1, at least 2, at least 3, at least 4, at least 5, at least 6, at least 7, at least 8, at least 9, at least 10, at least 11, at least 12, at least 13, at least 14 or all are conservative substitutions of the amino acids found in the corresponding positions in the sequence RASESVDNYGISFMN (SEQ ID NO: 4).

In another embodiment, the VL of the antibody of the invention is characterized in that the CDR2 region
- shows at least 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence AASNQGS (SEQ ID NO: 5);
- at least 1, at least 2, at least 3, at least 4, at least 5, at least 7 or all are conservative substitutions of the amino acids found in the corresponding positions in the sequence AASNQGS (SEQ ID NO: 5).

In another embodiment, the VL of the antibody of the invention is characterized in that the CDR 3 region
- shows at least 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity with the sequence QQSKEVPWT (SEQ ID NO: 6);
- at least 1, at least 2, at least 3, at least 4, at least 5, at least 6, at least 7, at least 8 or all are conservative substitutions of the amino acids found in the corresponding positions in the sequence QQSKEVPWT (SEQ ID NO: 6).

In a particular embodiment, the antibody of the invention comprises framework regions (FR), wherein the FR1, FR2, FR3 and FR4 of the VH region comprise respectively the sequences of SEQ ID NO: 7, 8, 9 and 10 or functionally equivalent variants thereof and/or wherein the FR1, FR2, FR3 and FR4 of the VL region comprise respectively the sequences of SEQ ID NO: 11, 12, 13 and 14 or functionally equivalent variants thereof.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1 (L1)-FR2-H2(L2)-FR3-H3 (L3)-FR4.

As it is used herein, the term "functionally equivalent variant of a FR sequence" refers to a sequence variant of a particular FR sequence having substantially similar sequence identity with it and substantially maintaining its capacity to bind to its cognate antigen when being part of an antibody or antibody-binding domains described herein. For example, a functionally equivalent variant of a FR sequence may be a polypeptide sequence derivative of said sequence comprising the addition, deletion or substitution of one or more amino acids.

Functionally equivalent variants of a FR sequence according to the invention include FR sequences having at least approximately 70%, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a FR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a FR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 7-10 and 11-14 to bind to its cognate antigen when being part of an antigen-binding domain of the invention. This capacity to bind to its cognate antigen may be determined as a value of affinity, avidity, specificity and/or selectivity of the antibody or antibody fragment to its cognate antigen.

In another particular embodiment, the VH region of the antibody of the invention comprises the sequence of SEQ ID NO: 15 and the VL region of the antibody of the invention comprises the sequence of SEQ ID NO: 16.

In another particular embodiment, the VH region of the antibody of the invention comprises the sequence of SEQ ID NO: 15 or a functionally equivalent variant thereof and the VL region of the antibody of the invention comprises the sequence of SEQ ID NO: 16 or a functionally equivalent variant thereof.

In a particular embodiment, the VH of the antibody of the invention comprises the sequence having at least 75%, at least 80%, 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 15.

In a particular embodiment, the VL of the antibody of the invention comprises sequences having at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with SEQ ID NO: 16.

In a further embodiment, the antibody of the invention comprises at least one framework region derived from a human antibody framework region.

In one embodiment, the VH region of the antibody of the invention comprises at least one humanized FR region, at least 2 humanized FR regions, at least 3 humanized FR regions or at least 4 humanized FR regions.

In another embodiment, the VL region of the antibody of the invention comprises at least one humanized FR region, at least 2 humanized FR regions, at least 3 humanized FR regions or at least 4 humanized FR regions.

In further embodiments, the antibody of the invention is humanised or super-humanised.

By "humanised" is meant an antibody derived from a non-human antibody, typically a murine antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Methods for humanizing non-human antibodies have been described in the art. Preferably, a humanised antibody has one or more amino acid residues introduced into it from a source which is non-human. These non-human amino acid residues are often referred to as "import" residues, which are typically taken from an "import" variable domain. Humanisation can be essentially performed following the method of Winter and co-workers (Jones et al., Nature, 321 :522-525 (1986); Reichmann et al., Nature, 332:323-327 (1988); Verhoeyen et al., Science, 239: 1534-1536 (1988)), by substituting hypervariable region sequences for the corresponding sequences of a human antibody. In practice, humanised antibodies are typically human antibodies in which some hypervariable region residues and possibly some framework region (FR) residues are substituted by residues from analogous sites in rodent antibodies. The choice of human variable domains, both light and heavy, to be used in making the humanised antibodies is very important to reduce immunogenicity retaining the specificity and affinity for the antigen. According to the so called "best-fit" method, the sequence of the variable domain of a rodent antibody is screened against the entire library of known human variable-domain sequences. The human sequence which is closest to that of the rodent is then accepted as the human framework region (FR) for the humanised antibody (Suns et al., J. Immunol, 151 :2296 (1993)). Another method uses a particular framework region derived from the consensus sequence of all human antibodies of a particular subgroup of light or heavy chains. The same framework may be used for several different humanised antibodies (et al., J. Immunol, 151 :2623 (1993)).

The term "super-humanised" refers to humanized antibodies in which there is a transfer of murine CDRs onto human germline framework regions defining the same canonical structure classes of the hypervariable loops and based on highest CDR sequence homology as described in P. Tan et al., J Immunol Baltim Md 1950 169 (2002), 1119-1125 and W.Y. Khee Hwang et al., Methods 36 (2005), 35-42.

It is further important that antibodies are humanised with retention of high affinity for the antigen and other favorable biological properties. To achieve this goal, humanised antibodies are prepared by a process of analysis of the parental sequences and various conceptual humanised products using three-dimensional models of the parental and humanised sequences. A further step in this approach, to make an antibody more similar to humans, is to prepare the so called primatised antibodies, i.e. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant).

By "human antibody" is meant an antibody containing entirely human light and heavy chains as well as constant regions, produced by any of the known standard methods.

As an alternative to humanisation, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH gene in chimeric and germ-line mutant mice results in the complete inhibition of endogenous antibody production. Transfer of the human germ-line immunoglobulin gene array in such germ line mutant mice will result in the production of human antibodies after immunization. See, e.g., Lonberg, 2005, Nature Biotech. 23: 1117-25.

Human antibodies may also be generated by in vitro activated B cells or SCID mice with its immune system reconstituted with human cells.

Once a human antibody is obtained, its coding DNA sequences can be isolated, cloned and introduced into an appropriate expression system, i.e., a cell line, preferably from a mammal, which subsequently express and liberate it into a culture media from which the antibody can be isolated.

In another preferred embodiment, the antibody of the invention is a Fab, a F(ab)₂, a single-domain antibody, a single chain variable fragment (scFv), or a nanobody. Fab, F(ab)2, single-domain antibodies, single chain variable fragments (scFv), and nanobodies are considered epitope-binding antibody fragments.

An antibody fragment is a fragment of an antibody such as, for example, Fab, F(ab')₂, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific.

Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind the antigen, although with lower affinity than the entire binding site.

The Fab fragment also contains the constant domain of the light chain and the first constant domain (CHI) of the heavy chain. Fab' fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known.

The term "single domain antibodies" makes reference to antibodies whose complementary determining regions are part of a single domain polypeptide. Examples include, but are not limited to, heavy chain antibodies (nanobodies), antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine.

"Single-chain Fv" or "scFv" antibody fragments comprise the V_{H} and VL domains of an antibody, wherein these domains are present in a single polypeptide chain. Preferably, the Fv polypeptide further comprises a polypeptide linker between the VH and VL domains which enables the scFv to form the desired structure for antigen binding.

More preferably, although the two domains of the Fv fragment, VL and VH, are naturally encoded by separate genes, or polynucleotides that encode such gene sequences (e.g., their encoding cDNA) can be joined, using recombinant methods, by a flexible linker that enables them to be made as a single protein chain in which the VL and VH regions associate to form monovalent epitope-binding molecules (known as single-chain Fv (scFv). Alternatively, by employing a flexible linker that is too short (e.g., less than about 9 residues) to enable the VL and VH domains of a single polypeptide chain to associate together, one can form a bispecific antibody, diabody, or similar molecule (in which two such polypeptide chains associate together to form a bivalent epitope-binding molecule. Examples of epitope-binding antibody fragments encompassed within the present invention include (i) a Fab' or Fab fragment, a monovalent fragment consisting of the VL, VH, CL and CH1 domains, or a monovalent antibody as described in WO2007059782; (ii) F(ab')2 fragments, bivalent fragments comprising two Fab fragments linked by a disulfide bridge at the hinge domain; (iii) an Fd fragment consisting essentially of the VH and CH1 domains; (iv) a Fv fragment consisting essentially of a VL and VH domains, (v) a dAb fragment, which consists essentially of a VH domain and also called domain antibodies; (vi) camelid or nanobodies and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH domains pair to form monovalent molecules (known as single chain antibodies or sisngle chain Fv (scFv).

The term "nanobodies" designates small sized entities (15 kDa) formed solely by the antigen binding region of the heavy chain (VH fragment) of immunoglobulins. Said nanobodies are mainly produced after immunizing animals of the Camelidae family, such as camels, llamas and dromedaries, mainly llamas; and also of the shark family, which have the particularity of having antibodies which naturally lack the light chain and recognize the antigen by the heavy chain variable domain. Nevertheless, the nanobodies derived from these sources require a humanization process for their therapeutic application. Another potential source for obtaining nanobodies is from antibodies derived from different human samples by separating the VH and VL domains of the variable region. Nanobodies present advantages such as a production cost reduction with respect to whole antibodies, stability and the reduction of immunogenicity.

The term "diabodies" refers to small antibody fragments with two antigen-binding sites, those fragments comprising a heavy-chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites.

Functional fragments of antibodies which bind to the RBD of the SARS-CoV-2 spike protein included within the present invention retain at least one binding function and/or modulation function of the full-length antibody from which they are derived. Preferred functional fragments retain an antigen-binding function of a corresponding full-length antibody (e.g., the ability to bind a mammalian CCR9).

The invention may also include bispecific antibodies. Bispecific antibodies are antibodies that have binding specificities for at least two different epitopes. Exemplary bispecific antibodies may bind to two different epitopes of the RBD of the SARS-CoV-2 spike protein. Bispecific antibodies can be prepared as full-length antibodies or antibody fragments (e.g. F(ab)₂ bispecific antibodies, minibodies, diabodies). According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

Techniques for generating bispecific antibodies from antibody fragments have also been described in the literature. For example, bispecific antibodies can be prepared using chemical linkage.

The fragments with the capacity to bind to the RBD of the SARS-CoV-2 spike protein or a functionally equivalent variant thereof can also be obtained by conventional methods known by persons having ordinary skill in the art. Said methods can involve isolating DNA that encodes the polypeptide chains (or a fragment thereof) of a monoclonal antibody of interest and manipulating DNA by means of recombinant DNA technology. DNA can be used to generate another DNA of interest, or an altered DNA, (for example by means of mutagenesis) for adding, removing or substituting one or more amino acids, for example, the DNA that encodes the polypeptide chains of an antibody (e.g., the heavy or light chains, the variable region or the whole antibody) can be isolated from murine B cells from immunized mice with the RBD of the SARS-CoV-2 spike protein. The DNA can be isolated and amplified by conventional methods, for example by means of PCR.

The single-chain antibodies can be obtained by conventional methods by binding the variable region of the heavy and light chains (Fv region) by means of an amino acid bridge. The scFvs can be prepared by fusing the DNA encoding a linker peptide between the DNAs encoding the polypeptides of the variable regions (V_{L} and V_{H}). The production of scFvs is described in a number of documents, for example, in US patent US 4,946,778

In another embodiment, the antibody comprises a VL domain and a VH domain. The term "VH domain" refers to the amino terminal variable domain of an immunoglobulin heavy chain, and the term "VL domain" refers to the amino terminal variable domain of an immunoglobulin light chain. The VL domain described herein may be linked to a constant domain to form a light chain, e.g., a full-length light chain. The VH domain described herein may be linked to a constant domain to form a heavy chain, e.g., a full-length heavy chain.

In a particular embodiment, the antibody of the invention is capable of neutralizing infection of mammalian cells by the SARS-CoV-2 virus with a 50% neutralization titer (NT50) of 0.07 nM or lower.

In a particular embodiment, the antibody of the invention is capable of neutralizing infection of Vero E6 cells by the SARS-CoV-2 virus (Wuhan) with a 50% neutralization titer (NT50) of 0,07 nM or lower.

The term "neutralizing" or "neutralizing antibody" refers to an antigen binding protein or antibody, respectively, that binds to a ligand and prevents or reduces the biological effect of that ligand. This can be done, for example, by directly blocking a binding site on the ligand or by binding to the ligand and altering the ligand's ability to bind through indirect means (such as structural or energetic alterations in the ligand). In some embodiments, the term can also denote an antigen binding protein that prevents the protein to which it is bound from performing a biological function. In assessing the binding and/or specificity of an antigen binding protein, e.g., an antibody or immunologically functional fragment thereof, an antibody or fragment can substantially inhibit binding of a ligand to its binding partner when an excess of antibody reduces the quantity of binding partner bound to the ligand by at least about 1-20, 20-30%, 30-40%, 40-50%, 50-60%, 60-70%, 70-80%, 80-85%, 85-90%, 90-95%, 95-97%, 97-98%, 98-99% or more (as measured in an in vitro competitive binding assay). In some embodiments, in the case of the RBD of the SARS-CoV-2 spike protein, such a neutralizing antibody can diminish the ability of RBD of the SARS-CoV-2 to bind the ACE2 receptor. In some embodiments, the neutralizing ability is characterized and/or described via a competition assay. In some embodiments, the neutralizing ability is described in terms of an IC 50 or EC 50 value. In some embodiments, the antibodies or antigen binding proteins neutralize by binding to the RBD of the SARS-CoV-2 spike protein and preventing the virus from binding to ACE2 receptor (or reducing the ability of the SARS-CoV-2 spike protein to bind to ACE2 receptor). In some embodiments, the antibodies neutralize by binding to the RBD of the SARS-CoV-2 spike protein, and while still allowing RBD of the SARS-CoV-2 spike protein to bind to ACE2 receptor, preventing or reducing the entering of the virus into the target cells. The term "NT 50" or "half maximal neutralizing concentration" refers to the concentration the antibody which induces a viral neutralizing response halfway between the baseline and maximum after a specified exposure time. More simply, NT50 can be defined as the concentration required to obtain a 50% of the viral neutralization effect.

The term "NT50" or "half maximal neutralizing concentration" refers to a measure of the potency of a substance (i.e. an antibody) in inhibiting a specific biological or biochemical function. NT50 is a quantitative measure that indicates how much of a particular inhibitory substance (an antibody in this particular case) is needed to inhibit, in vitro, a given biological process (i.e. inhibition of viral infection) or biological component by 50%.

The term "epitope" has already been described within the context of the first antibody of the invention and applies equally to the present case. In the present case, the epitope is a "conformational epitope", which refers to epitopes formed by the three-dimensional shape of the antigen (e.g., folding). The length of the epitope defining sequence can be subject to wide variations. Thus, the amino acids defining the epitope can be relatively few in number, but widely dispersed along the length of the molecule (or even on different molecules in the case of dimers, etc.), being brought into the correct epitope conformation via folding. The portions of the antigen between the residues defining the epitope may not be critical to the conformational structure of the epitope. For example, deletion or substitution of these intervening sequences may not affect the conformational epitope provided sequences critical to epitope conformation are maintained (e.g., cysteines involved in disulfide bonding, glycosylation sites, etc.)

### Polynucleotides, vectors and host cells

Polynucleotide sequences encoding monoclonal antibodies or fragments thereof, having high affinity and specificity for the RBD of the SARS-CoV-2 spike protein, as well as vectors and host cells carrying these polynucleotide sequences, are provided according to another aspect of the present invention

Therefore, in a second aspect, the invention relates to polynucleotides encoding the antibody according to the first aspect of the invention.

The present invention provides nucleic acid molecules, specifically polynucleotides which, in some embodiments, encode the antibody of the invention. The term "nucleic acid," in its broadest sense, includes any compound and/or substance that comprise a polymer of nucleotides. These polymers are often referred to as polynucleotides. The term "polynucleotide" as referred to herein means a polymeric form of nucleotides of at least 10 bases in length. In certain embodiments, the bases may be ribonucleotides or deoxyribonucleotides or a modified form of either type of nucleotide. The term includes single and double stranded forms of DNA.

Exemplary nucleic acids or polynucleotides of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, a-LNA having an a-L- ribo configuration (a diastereomer of LNA), 2'-amino-LNA having a 2 '-amino functionalization, and 2'-amino- a-LNA having a 2'-amino functionalization), ethylene nucleic acids (ENA), cyclohexenyl nucleic acids (CeNA) or hybrids or combinations thereof.

In one embodiment, linear polynucleotides encoding one or more antibody constructs of the present invention which are made using only in vitro transcription (IVT) enzymatic synthesis methods are referred to as "IVT polynucleotides." Methods of making IVT polynucleotides are known in the art and are described in co-pending International Publication No. WO2013151666 filed March 9, 2013 (Attorney Docket Number M300), the contents of which are incorporated herein by reference in their entirety.

Any of the polynucleotides described above may further include additional nucleic acids, encoding, e.g. a signal peptide to direct secretion of the encoded polypeptide, antibody constant regions as described herein, or other heterologous polypeptides as described herein. Also, as described in more detail elsewhere herein, the present invention includes compositions comprising one or more of the polynucleotides described above.

In one embodiment, the invention includes compositions comprising a first polynucleotide and a second polynucleotide wherein said first polynucleotide encodes a VH domain as described herein and wherein said second polynucleotide encodes a VL domain as described herein.

In a particular embodiment, the polynucleotide of the invention encodes the sequence of SEQ ID NO: 15 and SEQ ID NO: 16.

In a particular embodiment, the nucleic acid further comprises a sequence that encodes a signal peptide which is fused in frame at the N-terminus of the VH and/or the VL chain. The present invention also includes fragments of the polynucleotides of the invention. Additionally, polynucleotides that encode fusion polypeptides, Fab fragments, and other derivatives, as described herein, are also contemplated by the invention.

In some embodiments, the polynucleotide of the invention comprises, consist or essentially consists of the sequence of SEQ ID NO: 32 or SEQ ID NO:33.

In a particular embodiment, the polynucleotide of the invention encodes the sequence of SEQ ID NO: 34 and SEQ ID NO: 35.

The polynucleotides may be produced or manufactured by any method known in the art. For example, if the nucleotide sequence of the antibody is known, a polynucleotide encoding the antibody may be assembled from chemically synthesized oligonucleotides, which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding the antibody of the invention, variant, or derivative thereof of the invention, may be generated from nucleic acid from a suitable source. If a clone containing a nucleic acid encoding antibody of the invention is not available, but the sequence of the antibody molecules are known, a nucleic acid encoding the antibody may be chemically synthesized or obtained from a suitable source (e.g., an antibody cDNA library, or a cDNA library generated from, or nucleic acid, preferably poly A+RNA, isolated from, any tissue or cells expressing any of the antibodies , such as hybridoma cells selected to express an antibody) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibodies. Amplified nucleic acids generated by PCR may then be cloned into replicable cloning vectors using any method well known in the art.

Once the nucleotide sequence and corresponding amino acid sequence of the antibody thereof is determined, its nucleotide sequence may be manipulated using methods well known in the art for the manipulation of nucleotide sequences, e.g., recombinant DNA techniques, site directed mutagenesis, PCR, etc. (see, for example, the techniques described in Sambrook et al. (1990) Molecular Cloning, A Laboratory Manual (2nd ed.; Cold Spring Harbor Laboratory, Cold Spring Harbor, N.Y.) and Ausubel et al., eds. (1998) Current Protocols in Molecular Biology (John Wiley & Sons, NY), to generate antibodies having a different amino acid sequence, for example to create amino acid substitutions, deletions, and/or insertions.

A polynucleotide encoding the antibody, or antigen-binding fragment, variant, or derivative thereof, can be composed of any polyribonucleotide or polydeoxyribonucleotide, which may be unmodified RNA or DNA or modified RNA or DNA. For example, a polynucleotide encoding the antibody of the invention, or antigen-binding fragment, variant, or derivative thereof can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, a polynucleotide encoding any o the antibody of the invention can be composed of triple-stranded regions comprising RNA or DNA or both RNA and DNA.

A polynucleotide encoding the antibody of the invention may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

An isolated polynucleotide encoding a non-natural variant of a polypeptide derived from an immunoglobulin (e.g., an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCRmediated mutagenesis. Preferably, conservative amino acid substitutions are made at one or more non-essential amino acid residues.

In one embodiment, the polynucleotides have a modular design to encode the antibody, fragments or variants thereof described herein. As a non- limiting example, the polynucleotide construct may encode any of the following designs: (1) the heavy chain of an antibody, (2) the light chain of an antibody, (3) the heavy and light chain of the antibody, (4) the heavy chain and light chain separated by a linker, (5) the VH1, CH1, CH2, CH3 domains, a linker and the light chain and (6) the VH1 CH1, CH2, CH3 domains, VL region, and the light chain. Any of these designs may also comprise optional linkers between any domain and/or region.

In a particular embodiment the polynucleotide of the invention encodes a Fab, a F(ab)₂, a single domain antibody, a single chain variable fragment (scFv), or a nanobody.

In a related aspect the invention relates to an expression vector comprising the polynucleotide encoding the antibody of the invention

"Vector" includes shuttle and expression vectors and includes, e.g., a plasmid, cosmid, or phagemid. Typically, a plasmid construct will also include an origin of replication (e.g., the ColE1 origin of replication) and a selectable marker (e.g., ampicillin or tetracycline resistance), for replication and selection, respectively, of the plasmids in bacteria. An "expression vector" refers to a vector that contains the necessary control sequences or regulatory elements for expression of the antibody including antibody fragment of the invention, in prokaryotic, e.g., bacterial, or eukaryotic cells. Suitable vectors are disclosed below.

For recombinant production of the antibody, the nucleic acid molecule encoding it is isolated and inserted into a replicable vector for further cloning (amplification of the DNA) or inserted into a vector in operable linkage with a promoter for expression. DNA encoding the antibodies is readily isolated and sequenced using conventional procedures (e.g., by using oligonucleotide probes that are capable of binding specifically to nucleic acid molecules encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence.

The antibody of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (i.e., cleaved by a signal peptidase) by the host cell. For prokaryotic host cells that do not recognize and process the native antibodies signal sequence, the signal sequence is substituted by a prokaryotic signal sequence selected, for example, from the group of the alkaline phosphatase, penicillinase, 1pp, or heat-stable enterotoxin II leaders. For yeast secretion the native signal sequence may be substituted by, e.g., the yeast invertase leader, oc factor leader (including *Saccharomyces* and *Kluyveromyces* cc-factor leaders), or acid phosphatase leader, the *C albicans* glucoamylase leader, or the signal described in WO 90/13646. In mammalian cell expression, mammalian signal sequences as well as viral secretory leaders, for example, the herpes simplex gD signal, are available. The DNA for such precursor region is ligated in reading frame to DNA encoding the antibody.

Both expression and cloning vectors contain a nucleic acid sequence that enables the vector to replicate in one or more selected host cells. Generally, in cloning vectors this sequence is one that enables the vector to replicate independently of the host chromosomal DNA and includes origins of replication or autonomously replicating sequences. Such sequences are well known for a variety of bacteria, yeast, and viruses. The origin of replication from the plasmid pBR322 is suitable for most Gram-negative bacteria, the 2 µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.* One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibodies nucleic acid, such as DHFR, thymidine kinase, metallothionein-I and -11, preferably primate metallothionein genes, adenosine deaminase, ornithine decarboxylase, etc. For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., ATCC CRL-9096).

Alternatively, host cells particularly wild-type hosts that contain endogenous DHFR, transformed or co-transformed with DNA sequences encoding the antibodies, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

A suitable selection gene for use in yeast is the trp1 gene present in the yeast plasmid YRp7. The trp1 gene provides a selection marker for a mutant strain of yeast lacking the ability to grow in tryptophan, for example, ATCC No. 44076 or PEP4. The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

In addition, vectors derived from the 1.6 pm circular plasmid pKDI can be used for transformation of *Kluyveromyces* yeasts. Alternatively, an expression system for largescale production of recombinant calf chymosin was reported for *K. lactis.* Van den Berg, Bio/Technology, 8:135 (1990). Stable multi-copy expression vectors for secretion of mature recombinant human serum albumin by industrial strains of *Kluyveromyces* have also been disclosed.

Expression and cloning vectors usually contain a promoter that is recognized by the host organism and is operably linked to the antibodies nucleic acid. Promoters suitable for use with prokaryotic hosts include the phoA promoter, P-lactamase and lactose promoter systems, alkaline phosphatase promoter, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the antibodies.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors. Examples of suitable promoter sequences for use with yeast hosts include the promoters for 3-phosphoglycerate kinase or other glycolytic enzymes, such as enolase, glyceraldehyde phosphate dehydrogenase, hexokinase, pyruvate decarboxylase, phosphofructokinase, glucose phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde phosphate dehydrogenase, and enzymes responsible for maltose and galactose utilization. Suitable vectors and promoters for use in yeast expression are further described in EP 73,657. Yeast enhancers also are advantageously used with yeast promoters.

Antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian, Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a Hindlll E restriction fragment A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human P-interferon cDNA in mouse cells under the control of a thymidine kinase promoter from herpes simplex virus. Alternatively, the Rous Sarcoma Virus long terminal repeat can be used as the promoter.

Transcription of a DNA encoding the antibody of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the antibodies-encoding sequences but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain nucleotide segments transcribed as polyadenylated fragments in the untranslated portion of the mRNA encoding antibodies. One useful transcription termination component is the bovine growth hormone polyadenylation region. See WO 94/11026 and the expression vector disclosed therein.

In another related aspect, the invention relates to a host cell comprising the polynucleotide or the vector of the invention.

The term "host cell", as used herein, refers to a cell into which a nucleic acid of the invention, such as a polynucleotide or a vector according to the invention, has been introduced and is capable of expressing the micropeptides of the invention. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B*. *subtilis* and *B*. *licheniformis* (e.g., *B. licheniformis* 41P disclosed in DD 266,710 published 12 Apr. 1989), *Pseudomonas* such as *P*. *aeruginosa,* and *Streptomyces.* One preferred *E. coli* cloning host is *E. coli* 294 (ATCC 31,446), although other strains such as *E. coli B*, *E. coli* X1776 (ATCC 31,537), and *E*. *coli* W31 10 (ATCC 27,325) are suitable. These examples are illustrative rather than limiting.

Full length antibody, antibody fragments, and antibody fusion proteins can be produced in bacteria, in particular when glycosylation and Fc effector function are not needed, such as when the therapeutic antibody is conjugated to a cytotoxic agent (e.g., a toxin) and the immunoconjugate by itself shows effectiveness in tumor cell destruction. Full length antibodies have greater half-life in circulation. Production in *E. coli* is faster and more cost efficient. For expression of antibody fragments and polypeptides in bacteria, see, e.g., U.S. Pat. No. 5,648,237 (Carter et. al.), U.S. Pat. No. 5,789,199 (Joly et al.), and U.S. Pat. No. 5,840,523 (Simmons et al.) which describes translation initiation region (TIR) and signal sequences for optimizing expression and secretion, these patents incorporated herein by reference. After expression, the antibody is isolated from the *E*. *coli* cell paste in a soluble fraction and can be purified through, e.g., a protein A or G column depending on the isotype. Final purification can be carried out similar to the process for purifying antibody expressed e.g, in CHO cells.

In addition to prokaryotes, eukaryotic microbes such as filamentous fungi or yeast are suitable cloning or expression hosts for antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, e.g., *K. lactis, K. fragilis* (ATCC 12,424), *K*. *bulgaricus* (ATCC 16,045), *K. wickeramii* (ATCC 24,178), *K. waltii* (ATCC 56,500), *K*. *drosophilarum* (ATCC 36,906), *K. thermotolerans,* and *K. marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, e.g., *Neurospora, Penicillium, Tolypocladium,* and *Aspergillus* hosts such as *A. nidulans* and *A. niger.*

Suitable host cells for the expression of glycosylated antibodies are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, *Arabidopsis* and tobacco can also be utilized as hosts. Cloning and expression vectors useful in the production of proteins in plant cell culture are known to those of skill in the art.

However, interest has been greatest in vertebrate cells, and propagation of vertebrate cells in culture (tissue culture) has become a routine procedure. Examples of useful mammalian host cell lines are monkey kidney CVI line transformed by SV40 (COS-7, ATCC CRL 1651); human embryonic kidney line; baby hamster kidney cells (BHK, ATCC CCL 10); Chinese hamster ovary cells/-DHFR, mouse sertoli cells, monkey kidney cells (CVI ATCC CCL 70), African green monkey kidney cells (VERO-76, ATCC CRL1587), human cervical carcinoma cells (HELA, ATCC CCL 2), canine kidney cells (MDCK, ATCC CCL 34), buffalo rat liver cells (BRL 3A, ATCC CRL 1442), human lung cells (W138, ATCC CCL 75), human liver cells (Hep G2, 1413 8065), mouse mammary tumor (MMT 060562, ATCC CCL5 1), TRI cells , MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

The host cells used to produce the antibody of this invention may be cultured in a variety of media. Commercially available media such as Ham's FIO (Sigma), Minimal Essential Medium (MEM)(Sigma), RPMI-1640 (Sigma), and Dulbecco's Modified Eagle's Medium (DMEM)(Sigma) are suitable for culturing the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibodies can be produced intracellularly, in the periplasmic space, or directly secreted into the medium. If the antibodies areproduced intracellularly, as a first step, the particulate debris, either host cells or lysed fragments, are removed, for example, by centrifugation or ultrafiltration.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains. Protein G is recommended for all mouse isotypes and for human γ3. The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX^{™}resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE^{™} chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (e.g., from about 0-0.25M salt).

### Medical uses

In a third aspect, the antibody, the nucleic acid, the expression vector or the host cell of the invention for use in medicine.

In one embodiment of the present invention, it is provided an antibody or antibody fragment according to the invention as herein described, nucleic acid, expression vector or host cells according to the invention for use in the treatment of a medical condition associated with a SARS Coronavirus, wherein the medical condition associated with a SARS Coronavirus is preferably COVID-19 or a SARS Coronavirus-associated respiratory disease.

In a fourth aspect, the invention relates to an antibody or antibody fragment according to the invention as herein described, nucleic acid, expression vector or host cells according to the invention for use in a method of treatment of a disease caused by a coronavirus comprising the RBD of the SARS-CoV-2 spike protein or an immunologically equivalent variant thereof.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "treatment" or "therapy" include arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

As used herein, the "patient" or "subject" may be a vertebrate. In the context of the present invention, the term "subject" includes both humans and animals, particularly mammals, and other organisms.

In particular, the treatment described herein relates to either reducing or inhibiting coronavirus infection or symptoms thereof via binding the viral Spike protein with the antibody or fragments thereof of the present invention. The prophylactic therapy as described herein is intended to encompass prevention or reduction of risk of coronavirus infection, due to a reduced likelihood of coronavirus infection of cells via interaction with the ACE2 protein after treatment with the antibody or fragments thereof described herein.

CoVs are a group of related viruses that cause diseases in mammals and birds. The scientific name for coronavirus is Orthocoronavirinae or Coronavirinae. Coronavirus belongs to the family *Coronaviridae.* The family is divided into *Coronavirinae* and *Torovirinae* sub-families, which are further divided into six genera: Alphacoronavirus, Betacoronavirus, Gammacoronavirus, Deltacoronavirus, Torovirus, and Bafinivirus. While viruses in the genera Alphacoronaviruses and Betacoronaviruses infect mostly mammals, the Gammacoronavirus infect avian species and members of the Deltacoronavirus genus have been found in both mammalian and avian hosts.

In humans, coronaviruses cause respiratory tract infections that can be mild, such as some cases of the common cold, and others that can be lethal, such as SARS, MERS, and COVID-19.

Various species of human coronaviruses are known, such as, without limitation, the a-CoVs Human coronavirus 229E (HCoV-229E) and Human coronavirus NL63 (HCoV-NL63), and the b-CoVs Human coronavirus OC43 (HCoV-OC43), Human coronavirus HKU1 (HCoV-HKU1), Middle East respiratory syndrome-related coronavirus (MERS-CoV), Severe acute respiratory syndrome coronavirus (SARS-CoV), and Severe acute respiratory syndrome coronavirus 2 (SARS-CoV-2),.

In some embodiments, the patient suffers from an infection, preferably with a SARS Coronavirus (SARS-CoV). As used herein, SARS Coronavirus refers to a Coronavirus that leads to severe acute respiratory syndrome (SARS). This syndrome is a viral respiratory disease of zoonotic origin that first surfaced in the early 2000s caused by the first-identified strain of the SARS coronavirus (SARS-CoV or SARS-CoV-1).

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

In an embodiment, the antibody, nucleic acid, vector or host cell of the invention is administered to a patient or subject that is at risk of developing a severe acute respiratory syndrome (SARS).

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a SARS has a coronavirus infection.

Embodiments of a SARS coronavirus include, without limitation, any coronavirus that induces a SARS or SARS-similar pathology. Particular embodiments include, without limitation, the SARS Coronavirus (SARS-CoV-1) first discovered in 2003 (as described above), the Middle East respiratory syndrome (MERS-CoV) first discovered in 2012, and the SARS-CoV-2, which causes COVID-19, a disease which brought about the 2019-2020 coronavirus pandemic.

The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain. While the majority of cases result in mild symptoms, some progress to viral pneumonia and multi-organ failure.

According to Jin et al (Viruses 2020, 12, 372), in the initial 41 patients, fever (98%), cough (76%), and myalgia or fatigue (44%) were the most common symptoms. Less common symptoms were sputum production (28%), headache (8%), hemoptysis (5%), and diarrhea (3%). More than half of patients developed dyspnea. The average incubation period and basic reproduction number (R0) were estimated to be 5.2 d (95% CI: 4.1-7.0) and 2.2 (95% CI, 1.4-3.9), respectively.

In some embodiments, subjects have been tested and determined to have a SARS-Cov. Various methods may be employed to detect SAR-CoV, e.g. SARS-CoV-2, such as a nucleic acid test, serologic diagnosis, CRISPR/Cas13-based SHERLOCK technology, imaging technology, such as chest radiographs or CT-scans, or by the diagnostic method disclosed herein below.

Multiple SARS-CoV-2 variants are circulating globally. Additional variants and mutants of SARS-CoV-2 are being discovered regularly. Additional variants have been described above within the context of the S protein and the SARS-CoV-2-Spike-S1-RBD regions and equally apply to the present case.

In embodiments of the invention the antibody or fragments described herein can bind and/or neutralize multiple SARS-CoV-2 variants, representing an unexpected and beneficial property of the invention.

The term "immunologically functional equivalent" or "immunologically equivalent variant" are used indistinctly herein and refer to a variant of a polypeptide that retains substantially equivalent ability to induce an immune response in a subject as the reference polypeptide. The term "immunologically functional equivalent" is well understood in the art and is further defined in detail herein. Immunologically functional equivalents may increase the antigenicity of a polypeptide, maintain the same level of antigenicity of the reference polypeptide, or decrease the antigenicity of a polypeptide only slightly so that it maintains its usefulness as an antigen in an immunogenic composition.

The term "substantially equivalent", as used herein, refers to variants which are able to generate an immune response which is differs from the immune response generated with the native region by no more than 5 %, no more than 10 %, no more than 15 %, no more than 20 %, no more than 25 %, no more than 30 %, no more than 35 %, no more than least 40 %, no more than 45 %, no more than 50 %, no more than 55 %, no more than 60 %, no more than 65 %, no more than 70 %, no more than 75 %, no more than 80 %, no more than 85 %, no more than 90 % or no more than 95 %.

Immunologically functional equivalents of the RBD of the SARS-CoV-2 spike protein according to the invention contain modifications of the polypeptide. Said modifications can be, without limitation, amino acid changes, deletions, truncations, polypeptide fragments, fusions to other polypeptides, insertions, or any combination thereof. Accordingly, immunologically functional equivalents of the RBD of the SARS-CoV-2 spike protein show a degree of identity with respect to the amino acid sequence shown of the corresponding native RBD of the SARS-CoV-2 spike protein of at least 50%, at least 55%, at least 60%, at least 65%, at least 70%, at least 75%, at least 80%, at least 85%, at least 90%, at least 95%, at least 96%, at least 97%, at least 98% or at least 99% provided the immunogenic activity of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art. The identity between two amino acid sequences of two peptides is preferably determined using the BLASTP algorithm. See Altschul, S. et al., "BLAST Manual", (NCBI NLM NIH Bethesda, Md. 20894, Altschul, S., et al., J., 1990, Mol. Biol. 215:403-410).

As an example of modifications contemplated to be within the scope of the present invention, certain amino acids may be substituted for other amino acids in a polypeptide structure without appreciable loss of interactive binding capacity of the structure such as, for example, the epitope of an antigen that is recognized and bound by an antibody. Since it is the interactive capacity and nature of a polypeptide that defines its biological (e.g. immunological) functional activity, certain amino acid sequence substitutions can be made in an amino acid sequence (or its underlying DNA coding sequence) and nevertheless obtain a polypeptide with comparable properties. Various changes may be made to the amino acid sequences of the antigens of the present invention without appreciable loss of immunogenic activity.

It is understood in the art that in functionally equivalent amino acid substitutions, the hydropathic index of amino acids may be considered. The importance of the hydropathic amino acid index in conferring interactive biological function on a polypeptide is generally understood in the art. See Kyte J, Doolittle R, J. Mol. Biol. 1982; 15(1):105-132. It is known that certain amino acids may be substituted for other amino acids having a similar hydropathic index or score and still retain a similar biological activity.

In a particular embodiment, the coronavirus comprising the RBD of the SARS-CoV-2 spike protein or an immunologically equivalent is selected from SARS-CoV-1 and SARS-CoV-2.

### Pharmaceutical compositions

In fifth aspect, the invention relates to a pharmaceutical composition comprising the antibody or fragment thereof of the invention, nucleic acids, expression vectors, host cells of the invention together with a pharmaceutically acceptable carrier. A pharmaceutically acceptable carrier in the sense of the present invention may be any non-toxic material that does not significantly interfere in a detrimental sense with the effectiveness of the biological activity of the antibody of the present invention.

Evidently, the characteristics of the carrier will depend on the route of administration. Such a composition may contain, in addition to the active substance and carrier, diluents, fillers, salts, buffers, stabilizers, solubilizers, and other materials well known in the art. Formulation of pharmaceutically-acceptable excipients and carrier solutions is well-known to those of skill in the art, as is the development of suitable dosing and treatment regimens for using the particular compositions described herein in a variety of treatment regimens, including, without limitation, oral, parenteral, intravenous, sub-cutaneous, intranasal, inhalation, and intramuscular administration.

The medicament, otherwise known as a pharmaceutical composition, containing the active ingredient (antibody or antibody fragment, nucleic acids, expression vectors of host cells) may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets.

These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated, or they may be coated by known techniques to delay disintegration and absorption in the gastrointestinal tract and thereby provide a sustained action over a longer period. For example, a time delay material such as glyceryl monostearate or glyceryl distearate may be employed. They may also be coated. The present invention also refers to a pharmaceutical composition for topical application, oral ingestion, inhalation, or cutaneous, subcutaneous, or intravenous injection. A skilled person is aware of the carriers and additives required for particular application forms.

The medicament, otherwise known as a pharmaceutical composition, containing the active ingredient (antibody or antibody fragment, nucleic acids, expression vectors, host cells) may be in a form suitable for injection. Modes of administration involving injection comprise, without limitation, Subcutaneous (under the skin), Intramuscular (in a muscle), Intravenous (in a vein) or Intrathecal (around the spinal cord). Antibody therapies are typically administered using intravenous administration, which is a preferred mode of administration of the present invention.

Excipients, as an example of pharmaceutically acceptable carrier, for liquid formulations intended for injection are known in the art and can be selected appropriately by a skilled person. Excipients have been used to increase the stability of a wide range of protein and peptide-based formulations by reducing protein dynamics and motion, increasing the conformational stability of mAbs especially at high concentrations and inhibiting interface-dependent aggregation. Excipients usually inhibit aggregation and protects the protein by adsorbing to the air-liquid interface; for example, the use of surfactants (e.g., polysorbate 20 and 80), carbohydrates (e.g., cyclodextrin derivatives) and amino acids (e.g., arginine and histidine) can help prevent aggregation by this mechanism. Cyclodextrin has been reported to stabilize commercially available antibody-based drugs in a hydrogel formulation. Some of the generally recognized as safe (GRAS) excipients include pluronic F68, trehalose, glycine and amino acids such as arginine, glycine, glutamate and histidine, which are found in a number of commercial protein therapeutic products. By way of example, bevacizumab, 25 mg/ml_, contains trehalose dehydrate, sodium phosphate and polysorbate 20. Excipients in subcutaneous trastuzumab, 600 mg, are rHuPH20, histidine hydrochloride, histidine, trehalose dehydrate, polysorbate 20, methionine and water for injection.

When a therapeutically effective amount of the active substance (antibody or antibody fragment, nucleic acids, expression vectors, host cells) of the invention is administered by intravenous, cutaneous or subcutaneous injection, the active substance may be in the form of a solution, preferably a pyrogen-free, parenterally acceptable aqueous solution.

The preparation of such parenterally acceptable solutions, having due regard to pH, isotonicity, stability, and the like, is within the skill in the art. A preferred pharmaceutical composition for intravenous, cutaneous, or subcutaneous injection should contain, in addition to the active substance, an isotonic vehicle such as Sodium Chloride Injection, Ringer's Injection, Dextrose Injection, Dextrose and Sodium Chloride Injection, Lactated Ringer's Injection, or another vehicle as known in the art. The pharmaceutical composition of the present invention may also contain stabilizers, preservatives, buffers, antioxidants, or other additives known to those of skill in the art.

The invention also relates to administration of a therapeutically relevant amount of antibody as described herein in the treatment of a subject who has the medical disorders as disclosed herein. As used herein, the term "therapeutically effective amount" means the total amount of each active component of the pharmaceutical composition or method that is sufficient to show a meaningful patient benefit. The amount of active substance in the pharmaceutical composition of the present invention will depend upon the nature and severity of the condition being treated, and on the nature of prior treatments which the patient has undergone. Larger doses may be administered until the optimal therapeutic effect is obtained for the patient, and at that point the dosage is not increased further.

The dose of the antibody administered evidently depends on numerous factors well-known in the art such as, e.g., the chemical nature and pharmaceutical formulation of the antibody, and of body weight, body surface, age and sex of the patient, as well as the time and route of administration. For an adult, the dose may exemplarily be between 0.001 pg and 1 g per day, preferably between 0.1 pg and 100 mg per day, more preferably between 1 pg and 100 mg per day, even more preferably between 5 pg and 10 mg per day. In a continuous infusion, the dose may exemplarily be between 0.01 pg and 100 mg, preferably between 1 pg and 10 mg per kilogram body mass per minute.

In another embodiment, the pharmaceutical composition additionally comprises a compound or composition suitable for the treatment of SARS-CoV-2 selected from the group consisting of an anti-RBD antibody, COVID-19 convalescent plasma, an immunosuppressant agent, a nucleoside analogue, a viral protease inhibitor or an anti-inflammatory molecule.

The term "composition", as used herein, relates to a material composition that comprises any of the above-mentioned antibodies or compounds in any proportion and quantity, as well as any product resulting, directly or indirectly, from the combination of the different antibodies or compounds in any quantity thereof.

In a preferred embodiment, wherein the additionally compound is an anti-RBD antibody, the w/w ratio of the antibodies forming part of the pharmaceutical composition of the invention is typically within the range from approximately 0.01:1 to 100:1. Suitable ratios include without limitation for example, 0.05:1, 0.1:1, 0.5:1, 1:1, 1:2, 1:3, 1:4, 1:5, 1:6, 1:7, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, 100:1, 95:1, 90:1, 85:1, 80:1, 75:1, 70:1, 65:1, 60:1, 55:1, 50, 45:1, 40:1, 35:1, 30:1, 25:1, 20:1, 15:1, 10:1, 9:1, 8:1, 7:1, 6:1, 5:1, 4:1, 3:1, 2:1, 1:0.5, 1:0.1 and 1:0.05.

In a still preferred embodiment, the w/w ratio of the antibodies forming part of the composition of the invention is 1:1.

Those skilled in the art will observe that the composition may be formulated as a single formulation or may be presented as separate formulations of each of the antibodies, which may be combined for joint use as a combined preparation. The composition may be a kit-of-parts wherein each of the components is individually formulated and packaged.

The term "anti-RBD antibodies" refers to commercially available antibodies that specifically bind to the RBD of the SARS-CoV-2 spike protein and which are different from the antibodies disclosed in the present application.

In a preferred embodiment, the anti-RBD antibody is selected from bamlanivimab, etesevimab, casirinimab, imdevimab, regdanvimab and sotrovimab.

The term "COVID-19 convalescent plasma" refers to the blood plasma of a person who has recovered from COVID-19, which contains antibodies to the infectious disease factor. For convalescent plasma therapy, the plasma can be transfused into an ill victim of COVID-19 as a treatment.

The term "Immunosuppressant agent" or "immunosuppressive agents", are drugs or compounds that inhibit or prevent activity of the immune system. Common examples of immunosuppressant agents are selected from glucocorticoids, cytostatics, antibodies, drugs acting on immunophilins or other drugs such as interferons, opioids, TNF binding proteins, mycophenolate or other small biological agents.

In another embodiment, the immunosuppressant agent is selected from dexamethasone and bariticinib.

Nucleoside analogs are synthetic, chemically modified nucleosides that mimic their physiological counterparts (endogenous nucleosides) and block cellular division or viral replication by impairment DNA/RNA synthesis or by inhibition of cellular or viral enzymes involved in nucleoside/tide metabolism. Nucleotide and nucleoside analogue inhibitors, hereafter abbreviated NI, are chemically synthesized analogues of purines and pyrimidines in which the heterocyclic ring or sugar moiety has been altered. Currently used to treat both chronic and acute viral infections, NIs are administered as nucleotide or nucleoside precursors or prodrugs, which are metabolized by host or viral kinases to their active triphosphate once inside the cell. NIs exert inhibitory effects on viral replication by one or more non-mutually exclusive mechanisms. First, mis-incorporation of foreign nucleotides in replicating viral genomes may cause chain termination and disrupt subsequent replication or transcription. Chain termination may be immediate (obligate) or may occur following a limited extent of continued RNA or DNA synthesis (non-obligate). Second, NIs may incorporate into elongating nucleotide chains, mispairing with and/or substituting natural nucleotides, thereby introducing mutations that potentially impair RNA synthesis, structure, or RNA-protein interactions or protein functions. Accumulation of mutations and loss of virus viability are referred to as lethal mutagenesis and error catastrophe. Through these mechanisms, NIs alter the genetic makeup of the virus, leading to a decrease in viral fitness with every consecutive replication cycle. Finally, NIs may cause depletion of pools of naturally occurring nucleotides by mimicking.

In another embodiment the nucleoside analogue is molnupiravir.

Protease inhibitors (Pis) are medications that act by interfering with enzymes that cleave proteins. Some of the most well-known are antiviral drugs widely used to treat HIV/AIDS and hepatitis C. These protease inhibitors prevent viral replication by selectively binding to viral proteases and blocking proteolytic cleavage of protein precursors that are necessary for the production of infectious viral particles.

In another embodiment, the viral protease inhibitor is selected from PF-00835231 and Paxlovid.

An "anti-inflammatory agent" as used herein is an agent that reduces inflammation. In some embodiments, an anti-inflammatory agent is selected from Carprofen and Celecoxib.

Within the context of the present invention, the pharmaceutical composition of the invention may comprise at least one of the additional components as mentioned above together with the antibody, nucleic acids, expression vectors of host cells of the invention or more than one of the mentioned additional components together with the antibody, nucleic acids, expression vectors of host cells of the invention.

In a sixth aspect, the invention relates to the use of the pharmaceutical composition of the invention or the pharmaceutical composition together with one or more of the additional referred compounds for use in the treatment of disease caused by SARS-CoV-2.

In an embodiment, the pharmaceutical composition of the invention is used in a method of treatment of a coronavirus comprising the RBD of the SARS-CoV-2 spike protein or an immunologically equivalent variant thereof.

The terms treatment, SARS-CoV-2 and immunologically equivalent variant have already been described within the context of the medical uses of the invention and equally apply to the use of the pharmaceutical composition of the invention.

### Method for detection of RBD of SARS-CoV-2 spike protein

In a seventh aspect the invention relates to a method of detecting the presence of the RBD of SARS-CoV-2 spike protein or an immunologically equivalent variant thereof in a sample, the method comprising:
a) contacting the sample with the antibody of the invention, wherein said contacting is carried out under non-reducing conditions; and
b) detecting the formation of a complex containing the antibody and the RBD of SARS-CoV-2 spike protein or an immunologically equivalent variant thereof,
wherein the detection of the complex indicates the presence of the RBD of SARS-CoV-2 spike protein or an immunologically equivalent variant thereof in the sample.

In general, the immunobinding methods include obtaining a sample suspected of containing the RBD of SARS-CoV-2 spike protein or an immunologically equivalent variant thereof and contacting the sample with a composition capable of selectively binding or detecting the the RBD of SARS-CoV-2 spike protein or an immunologically equivalent variant thereof, under conditions effective to allow the formation of immunocomplexes.

The sample may be any sample that is suspected of containing the the RBD of SARS-CoV-2 spike protein or an immunologically equivalent variant thereof, such as, for example, a tissue section or specimen, a homogenized tissue extract, a cell, an organelle, separated and/or purified forms of any of the above antigen-containing compositions, or any biological fluid, including blood, serum and plasma. Preferably, the sample suspected of containing the the RBD of SARS-CoV-2 spike protein or an immunologically equivalent variant thereof is blood, serum or plasma.

Contacting the chosen biological sample with the antibody of the invention under effective conditions and for a period of time sufficient to allow the formation of immune complexes is generally a matter of simply adding the antibody composition to the sample and incubating the mixture for a period of time long enough for the antibodies to form immune complexes.

"Under conditions adequate for the formation of a complex" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background.

The "suitable" or "adequate" conditions also mean that the incubation is at a temperature or for a period of time sufficient to allow effective binding. Incubation steps are typically from about 1 to 2 to 4 hours or so, at temperatures preferably on the order of 25° C. to 27° C., or may be overnight at about 4° C. or so.

Non-reducing conditions refers to conditions wherein the interactions between two polypeptides are preserved, that is wherein disulfide bond tethers together two polypeptide chains. This usually requires not using beta-mercaptoethanol (2-ME) or dithiothreitol (DTT) to reduce disulphide bridges in proteins. This is used when certain antibodies cannot detect proteins in their reduced forms and in that case one uses non-reducing conditions, which means not using 2-ME or DTT in a gel loading buffer.

The determination of the amount of complex formed may be done in a number of ways. In a preferred embodiment, the antibody is labelled, and binding determined directly. For example, this may be done by attaching the the RBD of SARS-CoV-2 spike protein or an immunologically equivalent variant thereof to a solid support, adding the labelled antibody (for example a fluorescent label), washing off excess reagent, and determining whether the label is present on the solid support. Various blocking and washing steps may be utilized as is known in the art.

In a further embodiment, the antibody of the invention are coupled to a detectable label. In another preferred embodiment, said label can be detected by means of a change in at least one of its physical, chemical, electrical or magnetic properties.

In the context of the present invention, the expressions the "antibody is labelled", "labelled antibody", "detectable label" or "labelling agent", as used herein, refer to a molecular label which allows the detection, localization and/or identification of the molecule to which it is attached, using suitable procedures and equipment for detection, for example by spectroscopic, photochemical, biochemical, immunochemical or chemical means. Labelling agents that are suitable for labelling the antibodies include radionuclides, enzymes, fluorophores, chemiluminescent reagents, enzyme substrates or cofactors, enzyme inhibitors, particles, magnetic particles, dyes and derivatives, and the like.

The compounds radioactively labeled by means of radioactive isotopes, also called radioisotopes or radionuclides, may include, without limitation, ³H, ¹⁴C, ¹⁵N, ³⁵S, ⁹⁰Y, ⁹⁹Tc, ^{m}In, ¹²⁵I, ¹³¹I, ¹³³Xe, ¹¹¹Lu, ²¹¹At and ²¹³B. Radioisotope labelling is performed typically by using chelating ligands that are capable of complexing metal ions such as DOTA, DOTP, DOTMA, DTPA and TETA. Methods for conjugating radioisotopes to proteins are well known in the prior art..

In another particular embodiment, the antibody of the invention is labelled with a fluorescent group. The fluorescent group can be attached to the side chains of the amino acids directly or through a linking group. Methods for conjugating polypeptides fluorescent reagents are well known in the prior art.

Suitable reagents for labelling polypeptides, such as antibodies, with fluorescent groups include chemical groups which show ability to react with the various groups listed in the side chains of the proteins, including amino groups and thiol groups. Thus, chemical groups that can be used to modify the antibody according to the present invention include, without limitation, maleimide, haloacetyl, iodoacetamide succinimidyl ester (e.g. NHS, N-hydroxysuccinimide), isothiocyanate, sulfonyl chloride, 2,6-dichlorotriazinyl, pentafluorophenyl ester, phosphoramidite and the like. An example of suitable reactive functional group is N -hydroxysuccinimide ester (NHS) of a detectable group modified with a carboxyl group. Typically, the carboxyl group modifying the fluorescent compound is activated by the contacting of said compound with a carbodiimide reagent (for example, dicyclohexylcarbodiimide, diisopropylcarbodiimide, uranium or a reagent such as TSTU (0-(N-Succinimidyl)-N,N,N',N'-tetramethyluronium tetrafluoroborate), HBTU((0-benzotriazol- 1 -yl)-N,N,N',N'-tetramethyluronium hexafluorophosphate), or HATU (0-(7-azabenzotriazol-I-yl)-N,N,N',N'-tetramethyluronium hexafluorophosphate), an activator of the type of 1-hydroxybenzotriazole (HOBt) and N-hydroxysuccinimide to give the NHS ester of the label.

The fluorescent labels may include, without limitation, ethidium bromide, SYBR Green, fluorescein isothiocyanate (FITC), rhodamine tetramethyl isotiol (TRIT), 5-carboxyfluorescein, 6-carboxyfluorescein, fluorescein, HEX (6-carboxy-2',4,4',5',7,7'-hexachlorofluorescein), Oregon Green 488, Oregon Green 500, Oregon Green 514, Joe (6-carboxy-4',5'-dichloro-2',7'-dimethoxyfluorescein), 5-carboxy-2',4',5',7'-tetrachlorofluorescein,5-carboxyrhodamine, rhodamine, tetramethylrhodamine (Tamra), Rox (carboxy-X-rhodamine), R6G (rhodamine 6G), phthalocyanines, azometazinas, cyanines (Cy2,Cy3 and Cy5), Texas Red, Princeston Red, BODIPY FL-Br2, BODIPY 530/550, BODIPY TMR, BODIPY 558/568, BODIPY 564/570, BODIPY 576/589, BODIPY 581/591, BODIPY TR, BODIPY 630/650, BODIPY 650/665, DABCYL, eosin, erythrosin, ethidium bromide, green fluorescent protein (GFP) and its analogues, inorganic-based fluorescent semiconductor nanocrystals (Quantum Dot), fluorescent labels based onlanthanide such as Eu³⁺ and Sm³⁺ and the like, rhodamine, phosphoruslanthanides or FITC.

The enzymatic labels may include, without limitation, horseradish peroxidase, β-galactosidase, luciferase or alkaline phosphatase.

The preferred labeling include, but are not limited to, fluorescein, a phosphatase such as alkaline phosphatase, biotin, avidin, a peroxidase such as horseradish peroxidase and compounds related to biotin or compounds related to avidin (for example, streptavidin or ImmunoPure^{®} NeutrAvidin available from Pierce, Rockford, IL).

In another particular embodiment, the antibody of the invention are labelled by conjugation to a first member of a binding pair. In a preferred embodiment, this modification is covalent biotinylation. The term "biotinylation", as used herein, refers to the covalent attachment of biotin to a molecule (typically a protein). Biotinylation is performed using biotin reagents capable of conjugating to the side chain of the proteins, wherein said conjugation occurs primarily on the primary amino groups and thiol groups contained in the side chains of proteins. Suitable reagents for biotinylation of amino groups include molecules containing biotin and a group capable of reacting with amino groups such as succinimide esters, pentafluorophenyl ester or alkyl halides, wherein the biotin moiety and the reactive group separated by a spacer of any length.

In another particular embodiment, the antibody of the invention is labelled with metal ions such as gold (Au), including colloidal gold nanoparticles can be attached directly to the antibody via electrostatic interactions. In another particular embodiment, the colloidal gold nanoparticles are pre-coupled to biotin and can be covalently attached to the antibody.

In general, the detection of immunocomplex formation is well known in the art and may be achieved through the application of numerous approaches. These methods are generally based upon the detection of a label or marker, such as any of those radioactive, fluorescent, biological and enzymatic tags. U.S. Patents concerning the use of such labels include U.S. Pat. Nos. 3,817,837; 3,850,752; 3,939,350; 3,996,345; 4,277,437; 4,275,149 and 4,366,241. Of course, one may find additional advantages through the use of a secondary binding ligand such as a second antibody and/or a biotin/avidin ligand binding arrangement, as is known in the art.

In a still preferred embodiment, the antibody used in step (i) or the antibodies within the composition used in step (i) of the first method of the invention are immobilized.

As the person skilled in the art will understand that there is a wide range of conventional assays that can be used in the present invention which use an antibody of the invention that is not labelled (primary antibody) and an antibody of the invention that is labelled (secondary antibody); these techniques include Western blot or immunoblot, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical and immunohistochemical techniques, flow cytometry or multiplex detection techniques based on using protein microspheres, biochips or microarrays which include the antibody of the invention. Other ways of detecting and quantifying the RBD of SARS-CoV-2 spike protein or an immunologically equivalent variant thereof using the antibody of the invention include affinity chromatography techniques, ligand binding assays or lectin binding assays.

It will also be understood that antibodies that are not labelled need to be detected with an additional reagent, for example, a secondary antibody that is labelled, which will be labelled. This is particularly useful in order to increase the sensibility of the detection method, since it allows the signal to be amplified.

In addition, the detection of the antibody can also be carried out by detecting changes in the physical properties in the sample that occur as a result of the binding of the antibody to its cognate antigen. These assays include determining a transmission-related parameter in a sample, which are known in the art. The term "transmission-related parameter", as used herein, relates to a parameter indicating or correlating with the ratio of transmitted light versus incident light of a sample or to a parameter derived therefrom.

In an embodiment, a transmission-related parameter is determined by turbidimetry or by nephelometry.

Turbidimetry, as used herein, refers to the measurement of light-scattering species in solution by means of a decrease in intensity of the incident beam after it has passed through solution. For turbidimetric assays, the change in the amount of light absorbed (inverse of amount transmitted) can be related to the amount of agglutination which occurs. Hence, the amount of analyte (the species causing agglutination) in the sample can be easily determined.

Nephelometry, as used herein, refers to a technique for measuring the light-scattering species in solution by means of the light intensity at an angle away from the incident light passing through the sample. Nephelometric assays present an indirect method of measurement of the amount of analyte in a sample by measuring the amount of light scattered or reflected at a given angle (typically 90°) from the origin. In the presence of the protein antigen, the antibody reacts with the antigen, and a precipitation reaction begins. The measurement is taken early in this precipitation reaction time sequence. A quantitative value is obtained by comparison with a standard curve, which has been established previously. In order to increase the sensitivity of the detection, the antibody can be adsorbed or covalently attached to polymeric microspheres. In this way, a greater signal is produced with less reagent.

The detection method based on turbidimetry or nephelometry according to the present disclosure works with all known agglutination tests with and without microparticles enhancement. Typically used within the present disclosure is a "microparticle-enhanced light scattering agglutination tests" which is also called "particle-enhanced turbidimetric immunoassays" (PETIA). Agglutination-based immunoassays are routinely used in clinical diagnostics for the quantitation of serum proteins, therapeutic drugs and drugs of abuse on clinical chemistry analyzers, because they have the benefits of being quasihomogeneous assays which do not require any separation or wash step. To enhance the optical detection between the antigen to be detected and the specific antibody in the reaction mixture, the antibody may be linked to suitable particles. Thereby, the antigen reacts and agglutinates with the particles which are coated with antibody. With increasing amount of antibody, the agglutination and the size of the complexes are increasing, leading further to a change of light scattering.

In another embodiment, the binding of the antibody to its cognate antigen can be detected by Surface plasmon resonance (SPR).

As used herein, SPR refers to a phenomenon that the intensity of a reflected light decreases sharply at a particular angle of incidence (i.e., an angle of resonance) when a laser beam is irradiated to a metal thin film. SPR is a measurement method based on the phenomenon described above and is capable of assaying a substance adsorbed on the surface of the metal thin film, which is a sensor, with high sensitivity. According to the present invention, for example, the target substance in the sample can then be detected by immobilizing the antibody according to the present invention on the surface of the metal thin film beforehand, allowing the sample to pass through the surface of the metal thin film, and detecting the difference of the amount of the substance adsorbed on the surface of the metal thin film resulting from the binding of the antibody and the target antigen, between before and after the sample passes therethrough.

### Diagnostic method

In an eighth aspect, the invention relates to a method of diagnosing a coronovirus infection in a subject wherein the coronavirus comprises the RBD of the SARS-CoV-2 spike protein or an immunologically equivalent variant thereof, the method comprising detecting the presence of the RBD of the SARS-CoV-2 spike protein or an immunologically equivalent variant thereof in a sample from the subject by the method of the seventh aspect of the invention, wherein the detection of the peptide of the RBD of the SARS-CoV-2 spike protein or an immunologically equivalent variant thereof in the sample is indicative that the patient suffers an infection of a coronavirus which comprises the RBD of the SARS-CoV-2 spike protein or an immunologically equivalent variant thereof.

In the context of the present invention, the term "diagnosis" relates to the ability to discriminate between samples from patients with coronavirus infection and samples from individuals who have not suffered a coronavirus infection, when applied a method as disclosed herein. This detection as it is understood by one skilled in the art is not intended to be 100% correct for all the samples. However, it requires that a statistically significant number of samples analyzed are classified correctly. The amount that is statistically significant can be set by an expert in the field by using different statistical tools, for example, but not limited by the determination of confidence intervals, p value determination, Student's t test and discriminating function Fisher. Preferably, the confidence intervals are at least 90%, at least 95%, at least 97%, at least 98% or less than 99%. Preferably, the p value is less than 0.05, 0.01, 0.005 or 0.0001. Preferably, the present invention can correctly detect coronavirus infection in at least 60%, at least 70%, by at least 80%, or at least 90% of the subjects of a particular group or population tested.

The term "subject" or "individual" or "animal" or "patient" includes any subject, particularly a mammalian subject, for whom therapy is desired. Mammalian subjects include humans, domestic animals, farm animals, and zoo or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "sample" or "biological sample", as used herein, refers to biological material isolated from a subject. The biological sample contains any biological material suitable for detecting the RBD of the SARS-CoV-2 spike protein or an immunologically equivalent variant thereof. The sample can be isolated from any suitable tissue or biological fluid such as, for example blood, saliva, plasma, serum, urine, cerebrospinal liquid (CSF) or feces. In a particular embodiment of the invention, the sample is a tissue sample or a biofluid. In a more particular embodiment of the invention, the biofluid is selected from the group consisting of blood, serum or plasma.

Preferably, the sample which is used for the detection of the presence of the RBD of the SARS-CoV-2 spike protein or an immunologically equivalent variant thereof is the same type of sample used for determining the reference value in case that the determination is done in relative terms. By way of an example, if the determination of the presence of the RBD of the SARS-CoV-2 spike protein or an immunologically equivalent variant thereof is carried out in a plasma sample, then a plasma sample will also be used to determine the reference value. If the sample is a biofluid, then the reference sample will also be determined in the same type of biofluid, e.g. blood, serum, plasma, cerebrospinal fluid.

In an embodiment, within the context of the antibody, nucleic acid, expression vector or host cell for use according to the method of the seventh aspect of the invention or the method according to eighth aspect of the invention, the coronavirus is SARS-CoV-2 and is selected from the group consisting of the Wuhan strain, having a genome with GenBank accession number MN908947.3, the variant of concern (VOC) Beta (South Africa; B.1.351; GISAID clade GH/501Y.V2), the VOC Gamma (Brazil; P.1; GISAID clade GR/501Y.V3), the VOC CAL.20C (California; B.1427/B.1429; GISAID clade GH/452R.V1) and the VOC Omicron (B.1.1.529; GSAID clade, GRA).

In a final aspect, the invention relates to a diagnostic kit or a screening kit comprising the antibody of the invention.

The screening kit of the present invention comprises the antibody or fragments thereof of the invention This antibody or fragment thereof is including in the screening kit of the present invention as a reagent for measuring the presence of coronavirus comprising the RBD of SARS-CoV-2 spike protein or an immunologically equivalent variant thereof in a specimen collected from a test subject.

The screening kit of the present invention can be in any form as long as it comprises the aforementioned antibody or fragment thereof, and may comprise, in addition, various reagents and tools for the identification of the antigen-antibody complex (AgAb).

The invention will be described by way of the following examples which are to be considered as merely illustrative and not limitative of the scope of the invention.

### Further Aspects of the Invention

1. An antibody which specifically recognizes the receptor binding domain (RBD) of the SARS-CoV-2 spike protein, wherein said antibody comprises:
   - a heavy chain variable region (VH) wherein the CDR1, CDR2 and CDR3 of the VH comprise, respectively, the sequences of SEQ ID NO: 1, 2 and 3 or functionally equivalent variants thereof, and
   - a light chain variable region (VL) wherein the CDR1, CDR2 and CDR3 of the VL region comprise respectively, the sequences of SEQ ID NO: 4, 5, and 6 or functionally equivalent variants thereof.
2. The antibody according to aspect 1 wherein the FR1, FR2, FR3 and FR4 of the VH region comprise respectively the sequences of SEQ ID NO: 7, 8, 9 and 10 or functionally equivalent variants thereof and/or wherein the FR1, FR2, FR3 and FR4 of the VL region comprise respectively the sequences of SEQ ID NO: 11, 12, 13 and 14 or functionally equivalent variants thereof.
3. The antibody according to any of aspects 1 or 2, wherein the VH region comprises the sequence of SEQ ID NO: 15 and the VL region comprises the sequence of SEQ ID NO: 16.
4. The antibody according to any of aspects 1 to 3, which comprises at least one humanized framework region.
5. The antibody according to any of aspects 1 to 4, which is of the IgM class, kappa type.
6. The antibody according to any of aspects 1 to 5 which is coupled to a detectable label.
7. The antibody according to any of aspects 1 to 6 which is capable of neutralizing infection of mammalian cells by the SARS-CoV-2 virus with an NT50 of 0,07 nM or lower.
8. A nucleic acid encoding the VH and/or the VL of the antibody according to any of aspects 1 to 7.
9. The nucleic acid of aspect 8 which encodes the polypeptide of SEQ ID NO: 15 and/or the polypeptide of SEQ ID NO: 16 or which comprises the sequence of SEQ ID NO: 32 and/or the sequence of SEQ ID NO:33.
10. The nucleic acid of aspects 8 or 9 wherein the nucleic acid further comprises a sequence that encodes a signal peptide which is fused in frame at the N-terminus of the VH and/or the VL chain.
11. An expression vector comprising the nucleic acid of any of aspects 8 to 10.
12. A host cell comprising the nucleic acid of any of aspects 8 to 10 or the expression vector of aspect 16.
13. The antibody according to any of aspects 1 to 7, the nucleic acid of any of aspects 8 to 10, the expression vector of v11 or the host cell of aspect 12 for use in medicine.
14. The antibody according to any of aspects 1 to 7, the nucleic acid of any of aspects 8 to 10, the expression vector of aspect 11 or the host cell of aspect 12 for use in a method of prevention and/or treatment of an infection and/or a disease caused by a coronavirus comprising the RBD of the SARS-CoV-2 spike protein or an immunologically equivalent variant thereof.
15. The antibody, nucleic acid, expression vector or host cell for use according to aspect 14, wherein the coronavirus is selected from SARS-CoV-1 and SARS-CoV-2.
16. A pharmaceutical composition comprising an effective amount of the antibody according to any of claims 1 to 7, the nucleic acid of any of aspects 8 to 10, the expression vector of aspect 11 or the host cell of aspect 12 and a pharmaceutically-acceptable carrier.
17. The pharmaceutical composition according to aspect 16, additionally comprising a compound or composition suitable for the treatment of SARS-CoV-2 infection selected from the group consisting of an anti-SARS-CoV-2 antibody, COVID-19 convalescent plasma, an immunosuppressant agent, a nucleoside analogue and a viral protease inhibitor or an anti-inflammatory molecule.
18. The pharmaceutical composition according to aspect 17, wherein:
   - the antibody is selected from bamlanivimab, etesevimab, casirinimab, imdevimab, regdanvimab and sotrovimab;
   - the immunosuppressant agent is selected from dexamethasone and bariticinib;
   - the nucleoside analogue is molnupiravir;
   - the viral protease inhibitor is selected from PF-00835231 and Paxlovid, and
   - the anti-inflammatory molecule is selected from Carprofen and Celecoxib.
19. The pharmaceutical composition according to any of aspects 17 or 18 for use in the treatment of disease caused by SARS-CoV-2.
20. A method of detecting the presence of the RBD of SARS-CoV-2 spike protein or an immunologically equivalent variant thereof in a sample, the method comprising:
   a) contacting the sample with the antibody according to any of aspects 1 to 7, wherein said contacting is carried out under non-reducing conditions; and
   b) detecting the formation of a complex containing the antibody and the RBD of SARS-CoV-2 spike protein or an immunologically equivalent variant thereof,
   wherein the detection of the complex indicates the presence of the RBD of SARS-CoV-2 spike protein or an immunologically equivalent variant thereof in the sample.
21. A method of diagnosing a coronavirus infection in a subject, wherein the coronavirus comprises the RBD of the SARS-CoV-2 spike protein or an immunologically equivalent variant thereof, the method comprising detecting the presence of the RBD of the SARS-CoV-2 spike protein or an immunologically equivalent variant thereof in a sample from the subject by the method of aspect 20, wherein the detection of the RBD of the SARS-CoV-2 spike protein or immunologically equivalent thereof in the sample is indicative that the patient suffers an infection of a coronavirus which comprises the RBD of the SARS-CoV-2 spike protein or an immunologically equivalent variant thereof.
22. The antibody, nucleic acid, expression vector or host cell for use according to aspect 15 or the method according to aspect 21, wherein the coronavirus is SARS-CoV-2 and is selected from the group consisting of the Wuhan strain, having a genome with GenBank accession number MN908947.3 (18 March 2020) comprising SEQ ID NO: 17, the variant of concern (VOC) Beta (South Africa; B.1.351; GISAID clade GH/501Y.V2) (comprising SEQ ID NO: 20), the VOC Gamma (Brazil; P.1; GISAID clade GR/501Y.V3) (comprising SEQ ID NO: 25), the VOC CAL.20C (California; B.1.427/B.1.429; GISAID clade GH/452R.V1) (comprising SEQ ID NO: 21) and the VOC Omicron (B.1.1.529; GSAID clade, GRA) (comprising SEQ ID NO: 27).
23. A diagnostic kit or a screening kit comprising an antibody according to any of aspects 1 to 7.

### EXAMPLES

### Example 1. Expression and purification of a homodimer form of the RBD of the Spike protein of SARS-CoV-2 in insect cells

### Production of His-RBD protein from different SARS-CoV-2 variants

Genes optimized for insect cell expression comprising the DNA region encoding the receptor binding domain (RBD), present on the spike (S) protein from different SARS-CoV-2 variants (Wuhan) (SEQ ID NO: 17), Brazil (SEQ ID NO: 25), South Africa (SEQ ID NO: 27), and California /LA (SEQ ID NO: 21) were synthesized (Genscript). These recombinant genes encode for chimeric proteins formed by the signal peptide (MVSAIVLYVLLAAAAHSAFA) (SEQ ID NO: 30) of the *Autographa californica* nuclear polyhedrosis virus (AcNPV) protein gp67 (UniProtKB/Swiss-Prot Accession Number P17501), followed by nine histidine residues (HHHHHHHHH) (SEQ ID NO: 31) fused to the N-terminus of the RBD domain of the Spike protein (residues 319-541) from either of the above SARS-CoV-2 variants. These recombinant genes were cloned independently into the pFastBac1 expression vector (Thermo Fisher Scientific). The resulting plasmids were used to generate the corresponding recombinant baculovirus his-RBD (rBV his_RBD) by using the Bac-to-Bac technology (Thermo Fisher Scientific). Each of the his_RBD proteins were produced by infecting HighFive cells (Thermo Fisher Scientific) with either of the rBV_RBDs at a multiplicity of infection of 3 plaque forming units per cell (PFU/cell). Infected cultures were maintained in TC-100 medium (Thermo Fisher Scientific) supplemented with 1% FCS for 72 h. After this period, cell medium was harvested and clarified by centrifugation (4500xg for 10 min) and filtration through 0.45 µm filters. Protein purifications were achieved performed by immobilized metal affinity chromatography (IMAC) followed by gel filtration. IMAC was carried out using 5 ml nickel NTA agarose cartridges (Agarose Bead Technologies S.L.) at a flow rate of 1.5 ml/min. Retained proteins were eluted with a linear gradient up to 500 mM Imidazole in Trissaline buffer (pH 7.5). Fractions were analyzed by SDS-PAGE, and those containing the RBD polypeptide were pooled together and concentrated using Amicon Ultra-15 centrifugal units with a 10-kDa cutoff membrane (Millipore). A representative SDS-PAGE analysis of this IMAC purified proteins is shown in Figure 1.

As it shown in Figure 1, under non-reducing conditions (NR), RBD recombinant protein is produced mainly as a homodimer, although other forms of the protein can be observed, such as monomer, and oligomers (denoted with asterisks in the Figure). All these oligomeric forms are converted into monomer when β-mercaptoethanol is added (R). This conversion of dimers and oligomeric forms into monomers indicates that dimer forms, and higher molecular weight aggregates, might be hold by disulphide bonds.

The concentrated proteins after IMAC purification were loaded onto a Superdex 75 10/3lncrease gel filtration (GE Healthcare) equilibrated with PBS. RBD peak fractions corresponding to the dimer were analyzed by SDS-PAGE and pooled together. Purified proteins were aliquoted and kept at -20°C. Analysis of the four purified proteins by electrophoresis (SDS-PAGE) under non-reducing conditions, followed by Coomasie-blue staining is shown in Figure 2.

### Example 2. Generation of monoclonal antibodies (mAbs) against HIS RBD (Wuham) protein

### 2.1. Mice immunization

10 mice (6-week-old BALB/c females) were immunized (50 µg/mouse per dose), intraperitoneally (ip) with 3 doses of purified recombinant RBD (Wuham) protein, using Carbopol (1mg/mL) as adjuvant. As control, 10 mice were immunized with PBS/Carbopol. Doses were administered at 21-day intervals. 21 days after the last immunization, mice were bled, and their sera were individually analyzed for reactivity against RBD protein by ELISA assays. Result is shown in Figure 3.

As shown in Figure 3, mice immunized with RBD exhibited a considerably anti-RBD antibody response (white bars). The extremely low responses in mice inoculated with PBS (grey bars), indicate the specificity of the data.

### 2.2. Testing the inhibitory effect against SARS-Co V-2 of the mice sera

To test the inhibitory effect against SARS-CoV-2 of the mice sera, it was carried out a seroneutralization test. In this assay, dilutions of virus containing 100 plaque-forming units (PFUs) of SARS-CoV-2 (Wuhan) were incubated for 1 h at 37°C with 1:3 serial dilutions of: i) the sera of two mice (mouse 1 and 2) immunized with the RBD protein, ii) the serum of a mouse immunized with PBS (negative control), or iii) a commercial monoclonal antibody (Sino Biological, Cat. No. 40592-MM57), with a demonstrated neutralizing activity (650 µg/mL stock solution) as a positive control. Then, the serum/virus mixtures were added in duplicate over confluent monolayers of Vero E6 cells grown in 12-well plates. After 1 h of adsorption the inoculum was removed and a mixture of DMEM-2% FCS with agarose (0.6%) was added. At 3 days post-infection, cells were fixed with 10% formaldehyde, the medium was removed, the cells were stained with crystal violet, and viral plaques were counted. The reduction in the number of lysis plaques with respect to the negative control indicates the neutralization effect of the serum. The results, shown in Table I, demonstrate that immunization with the RBD recombinant protein induces anti-RBD antibodies with a neutralizing capacity against SARS-CoV-2 comparable to 650 µg/mL of purified monoclonal antibody 40592-MM57.

**TABLE I**

| | % **NEUTRALIZATION** | | | |
|---|---|---|---|---|
| **DILUTION** | **CONTROL +** | **MOUSE 1** | **MOUSE 2** | **CONTROL-** |
| 1:8 | 100 | 100 | 100 | 0 |
| 1:24 | 89 | 83 | 98 | 0 |
| 1:72 | 71 | 69 | 65 | 0 |
| 1:216 | 33 | 38 | 27 | 0 |
| 1:648 | 17 | 25 | 0 | 0 |

### 2.3. Generation of hybridomas secreting mAbs that recognize RBD from SARS-CoV-2

Two of the RBD-immunized mice showing the higher anti-RBD antibody response were boosted again (ip) with purified recombinant RBD (50 µg/mouse plus carbopol as adjuvant). Four days later, mice were euthanized, and spleens were removed aseptically and homogenized. The resulting splenocytes were washed three times with serum free medium, erythrocytes were lysed, and the resulting cell suspension were fused with murine P3X63-Ag8653 myeloma cells. Fusion was carried out following standard procedures. Briefly, splenocytes were fused with myeloma cells in a 5: 1 ratio, by adding dropwise a solution of PEG 4000 (50%). After washing with serum-free medium, cells were centrifuged (900rpm/5min), the supernatant was discarded, and the pelleted cells resuspended in 10 mL of DMEM medium containing 15% of inactivated foetal bovine serum (FBS). Viable hybridoma cells were counted by its staining with Trypan blue (50% viable cells were obtained), diluted in complete DMEM medium with 15% SFB (final concentration of 2×10⁶ cells/mL) and left in a 75 mL flask in a CO₂ incubator at 37°C O/N. Next day, hybridoma cells were removed from the flask, centrifuged, resuspended in selection media (5% of FBS in SFM with 1× hypoxanthine, aminopterin, and thymidine (HAT) (Sigma-Aldrich, St. Louis, MO, USA). Finally, hybridoma cells were seeded in 12 wells plates and incubated in a CO₂ incubator at 37°C O/N for two weeks. After that, when hybridoma colonies were clearly observed, the supernatant from each well was individually tested for detection of recombinant RBD in ELISA assays. From these experiments six supernatants from hybridomas were clearly positives: 2H10, 1C6, 2B6, 3C5, 5D4 and 6A9, that were initially selected.

From the different supernatant of hybridomas recognizing RBD, those with neutralizing viral activity were selected. For this, it was carried out a seroneutralization test similar to the one described in example 2.2. In this case, 100 plaque-forming units (PFUs) of SARS-CoV-2 (Wuhan) were incubated for 1 h at 37°C with 1:3 serial dilutions of: i) supernatant of the hybridoma selected in example 2.3., or ii) a commercial monoclonal antibody (Sino Biological, Cat. No. 40592-MM57), with a demonstrated neutralizing activity (650 µg/mL stock solution) as a positive control. Results are shown in Table II.

**TABLE II**

| | **% NEUTRALIZATION** | | | | | | |
|---|---|---|---|---|---|---|---|
| **DILUTION** | **CONTROL +** | **mAb 2H10** | **mAb 1C6** | **mAb 2B6** | **mAb3C5** | **mAb5D4** | **mAb 6A9** |
| **1:6** | 100 | 100 | 5 | 3,4 | 0 | 0 | 0 |
| **1:18** | 96,5 | 98 | 5 | 0 | 0 | 0 | 0 |
| **1:54** | 87,9 | 88 | 3 | 0 | 0 | 0 | 0 |
| **1:162** | 72,4 | 81 | 0 | 0 | 0 | 0 | 0 |
| **1:486** | 27,5 | 34 | 0 | 0 | 0 | 0 | 0 |
| **1:1458** | 14 | 18 | 0 | 0 | 0 | 0 | 0 |

As shown in Table II, supernatant from hybridoma 2H10 exhibited a strong viral neutralizing activity, comparable with the one showed by the positive control. Cells from hybridoma 2H10 were frozen and selected for future developments.

### 2.3. Clonal selection of hybridoma 2H10

A vial of 2H10 hybridoma was thawed, resuspended in Clonacell^{™}-HY Medium E (Stemcell), and hybridoma cells were seeded in 6 well plates and maintained during 48h in a CO₂ incubator. After that, hybridoma cells were seeded in 96/well plate by a limiting dilution procedure. Supernatant from wells containing a unique clon. This clonal selection was repeated twice. From this screening, clone 2H10E2 was the one showing a better response in ELISA, thus hybridoma clone 2H10E2 was chosen for further amplification and characterization.

### Example 3. Purification of the mAb 2H10E2

The clone expressing 2H10E2 mAb was amplified at high density in roller bottles using complete DMEM media supplemented with 10% FCS, non-essential amino acids, 4mM glutamine and Pen/Strep. Supernatant was collected when cell death reached 50%.

Collected supernatant was supplemented with 0.8 M (NH₄)₂SO₄ by slow stirring, filtered through a 0.45 µM filter to remove precipitates and loaded immediately on a HiTrap IgM column (Cytiva) at 1 ml/min. Unbound sample was removed with 20 mM Na₃PO₄, 0.8 M (NH₄)₂SO₄, pH 7.5 and eluted with 15 column volumes of 20 mM Na₃PO₄ pH 7.5. Eluted protein was monitored by SDS-PAGE and fractions containing the antibody were pooled together and concentrated using a concentrator with 100 kDa cutoff and run through Superose 6 10/300 column (Cytiva).

Purity of purified mAb 2H10E2 was analyzed by electrophoresis (SDS-PAGE) followed by Coomasie-blue staining (Figure 4).

### Example 4. Characterization of the mAb 2H10E2

### 4.1. mAb 2H10E2 only recognizes RBD under non-reducing conditions

First, mAb 2H10E2 was characterized by western-blot. For this, the monomeric and dimeric forms of recombinant RBD were separated by SDS-PAGE under reducing and non-reducing conditions, proteins transferred to a nitrocellulose membrane, and their recognition by the mAb was tested in a western-blot assay (Figure 5).

As it is shown in Fig. 5, panel B, mAb 2H10E2 recognizes only both dimer and monomer under non-reducing conditions, indicating that the epitope recognized by the mAb is a conformational epitope that might stabilized by disulphide bridge/s.

### 4.2. Determination of Immunoglobulin (Ig) isotype of mAb 2H10E2

Ig Isotype of mAb 2H10E2 was determined by using the Pierce TM Rapid Isotyping Kit following the vendor instructions. The result showed that mAb 2H10A is an IgM/Kappa.

### 4.3. mAb 2H10E2 recognizes RBD from at least four different variants of SARS-CoV-2

It was of interest to test if the mAb 2H10E2 was able to recognize RBD from different SARS-CoV-2 variants. For this, dimers of the four recombinant RBDs produced (Example 1) were tested is western-blot against mAb (Figure 6).

This result demonstrates that mAb 2H10E2 recognize under non-reducing conditions RBD from different SARS-CoV-2 variants, which indicates that the conformational epitope recognized by the mAb is well conserved among them.

### 4.4. mAb of 2H10E2 recognizes spike protein in SARS-CoV-2-infected cells

Once shown that mAb 2H10E2 can recoginize purified recombinant RBD protein, it was important to demonstrate whether the mAb was also able to recognize the complete Spike protein in the context of a viral infection. To this end, VERO E6 cells were infected with SARS-CoV-2 (Wuhan), and viral-infected cells were analyzed by western-blot (Figure 7). As it is shown in Figure 7, and in agreement with the above results (Figure 5), the mAb 2H10E2 only recognizes Spike (S) protein under non-reducing conditions, whereas a commercial antibody recognizes S protein at both, reducing and non-reducing conditions. These results confirms that the epitope recognized by the mAb is present also in the virus and is conformational.

### Example 5. mAb 2H10E2 neutralizes SARS-CoV-2 in vitro

To test the anti-SARS-CoV-2 activity of purified mAb 2H10E2, a seroneutralization test was carried out. Briefly, samples of virus containing 100 plaque-forming units (PFUs) of SARS-CoV-2 (Wuhan) were incubated for 1 h at 37°C with 1:3 serial dilutions of: i) mAb 2H10E2 (0,5 µg/µL stock solution), ii) the serum of a mouse immunized with PBS (negative control), or iii) a commercial monoclonal antibody (Sino Biological, Cat. No. 40592-MM57), with a demonstrated neutralizing activity (2 µg/mL stock solution) as a positive control. Then, the serum/virus mixtures were added in duplicate over confluent monolayers of Vero E6 cells grown in 12-well plates. After 1 h of adsorption the inoculum was removed and a mixture of DMEM-2% FCS with agarose (0.6%) was added. At 3 days post-infection, cells were fixed with 10% formaldehyde, the medium was removed, the cells were stained with crystal violet, and viral plaques were counted. The reduction in the number of lysis plaques with respect to the negative control indicates the degree of neutralization of the serum. The results, shown in Table III, demonstrate that mAb 2H10E2 induced a potent neutralizing capacity against SARS-CoV-2, much higher (about 90 times) than purified mAb 40592-MM57. NT50 for the positive control is approximately achieved at a dilution of 1:2187, which corresponds with a 6,4nM concentration of mAb. However, the 60% of neutralization is achieved whit a 1;6561 dilution of mAb 2H10E2, which corresponds with a 0,07 nM concentration of the mAb 2H10E2.

**TABLE III**

| | **% NEUTRALlZACIÓN** | | |
|---|---|---|---|
| **DILUCIÓN** | **Purified 2H10E2 (0,5 mg/ml)** | **Negative control** | **Control positivo (2 mg/ml)** |
| **1:27** | 100 | 0 | 100 |
| **1:81** | 100 | 0 | 100 |
| **1:243** | 92 | 0 | ^{.} 94 |
| **1:729** | 80 | 0 | 82 |
| **1:2187** | 68 | 0 | 45 |
| **1:6561** | 60 | 0 | 8 |
| **1:19683** | 0 | 0 | 0 |
| **1:59049** | 0 | 0 | 0 |

### Example 6. Determination of the mAb 2H10E2 DNA and protein sequences

The full length of the mAb 2H10E2 was sequenced from hybridoma cells. Sequencing and analysis were performed by Genscript Probio. The sequencing procedure was as follows: Total RNA was isolated from the hybridoma cells using the kit FastPure Cell/Tissue Total RNA Isolation Kit (Vazyme, Cat. No.: RC112) following the manufacturer's instructions. Total RNA was then reverse-transcribed into cDNA using either isotype-specific anti-sense primers or universal primers following the technical manual of SMARTScribe Reverse Transcriptase (TaKaRa, Cat. No.: 639536). Antibody fragments of heavy chain and light chain were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes. The consensus sequence was provided.

## Claims

1. An antibody which specifically recognizes the receptor binding domain (RBD) of the SARS-CoV-2 spike protein, wherein said antibody comprises:
- a heavy chain variable region (VH) wherein the CDR1, CDR2 and CDR3 of the VH comprise, respectively, the sequences of SEQ ID NO: 1, 2 and 3 or functionally equivalent variants thereof, and
- a light chain variable region (VL) wherein the CDR1, CDR2 and CDR3 of the VL region comprise respectively, the sequences of SEQ ID NO: 4, 5, and 6 or functionally equivalent variants thereof.

2. The antibody according to claim 1 wherein the FR1, FR2, FR3 and FR4 of the VH region comprise respectively the sequences of SEQ ID NO: 7, 8, 9 and 10 or functionally equivalent variants thereof and/or wherein the FR1, FR2, FR3 and FR4 of the VL region comprise respectively the sequences of SEQ ID NO: 11, 12, 13 and 14 or functionally equivalent variants thereof.

3. The antibody according to any of claims 1 or 2, wherein the VH region comprises the sequence of SEQ ID NO: 15 and the VL region comprises the sequence of SEQ ID NO: 16.

4. The antibody according to any of claims 1 to 3 which is capable of neutralizing infection of mammalian cells by the SARS-CoV-2 virus with an NT50 of 0,07 nM or lower.

5. A nucleic acid encoding the VH and/or the VL of the antibody according to any of claims 1 to 4.

6. The antibody according to any of claims 1 to 4 or the nucleic acid of claim 5 for use in medicine.

7. The antibody according to any of claims 1 to 4 or the nucleic acid according to claim 5 for use in a method of prevention and/or treatment of an infection and/or a disease caused by a coronavirus comprising the RBD of the SARS-CoV-2 spike protein or an immunologically equivalent variant thereof.

8. The antibody, nucleic acid, expression vector or host cell for use according to claim 6, wherein the coronavirus is selected from SARS-CoV-1 and SARS-CoV-2.

9. A pharmaceutical composition comprising an effective amount of the antibody according to any of claims 1 to 4 of the nucleic acid according to claim 5 and a pharmaceutically-acceptable carrier.

10. The pharmaceutical composition according to claim 9, additionally comprising a compound or composition suitable for the prevention and treatment of SARS-CoV-2 infection selected from the group consisting of an anti-SARS-CoV-2 antibody, COVID-19 convalescent plasma, an immunosuppressant agent, a nucleoside analogue and a viral protease inhibitor or an anti-inflammatory molecule.

11. The pharmaceutical composition according to claim 10, wherein:
- the antibody is selected from bamlanivimab, etesevimab, casirinimab, imdevimab, regdanvimab and sotrovimab;
- the immunosuppressant agent is selected from dexamethasone and bariticinib;
- the nucleoside analogue is molnupiravir;
- the viral protease inhibitor is selected from PF-00835231 and Paxlovid, and
- the anti-inflammatory molecule is selected from Carprofen and Celecoxib.

12. The pharmaceutical composition according to any of claims 10 or 11 for use in the treatment of disease caused by SARS-CoV-2.

13. A method of detecting the presence of the RBD of SARS-CoV-2 spike protein or an immunologically equivalent variant thereof in a sample, the method comprising:
a) contacting the sample with the antibody according to any of claims 1 to 4, wherein said contacting is carried out under non-reducing conditions; and
b) detecting the formation of a complex containing the antibody and the RBD of SARS-CoV-2 spike protein or an immunologically equivalent variant thereof,
wherein the detection of the complex indicates the presence of the RBD of SARS-CoV-2 spike protein or an immunologically equivalent variant thereof in the sample.

14. A method of diagnosing a coronavirus infection in a subject, wherein the coronavirus comprises the RBD of the SARS-CoV-2 spike protein or an immunologically equivalent variant thereof, the method comprising detecting the presence of the RBD of the SARS-CoV-2 spike protein or an immunologically equivalent variant thereof in a sample from the subject by the method of claim 13, wherein the detection of the RBD of the SARS-CoV-2 spike protein or immunologically equivalent thereof in the sample is indicative that the patient suffers an infection of a coronavirus which comprises the RBD of the SARS-CoV-2 spike protein or an immunologically equivalent variant thereof.

15. The antibody or nucleic acid for use according to claim 14 or the method according to claim 6, wherein the coronavirus is SARS-CoV-2 and is selected from the group consisting of the Wuhan strain, having a genome with GenBank accession number MN908947.3 (18 March 2020) (comprising SEQ ID NO: 17), the variant of concern (VOC) Beta (South Africa; B.1.351; GISAID clade GH/501Y.V2) (comprising SEQ ID NO: 20), the VOC Gamma (Brazil; P.1; GISAID clade GR/501Y.V3) (comprising SEQ ID NO: 25), the VOC CAL.20C (California; B.1.427/B.1.429; GISAID clade GH/452R.V1) (comprising SEQ ID NO: 21) and the VOC Omicron (B.1.1.529; GSAID clade, GRA) (comprising SEQ ID NO: 27).
